# EUROPÄISCHE PATENTSCHRIFT

(11) **EP 3 420 349 B1**
(45) Veröffentlichungstag und Bekanntmachung des Hinweises auf die Patenterteilung: **02.09.2020**
(21) Anmeldenummer: 17709011.5
(22) Anmeldetag: 23.02.2017
(51) Int. Cl.: G01N 33/24

(54) **VERFAHREN UND SCHALTUNGSANORDNUNG ZUM SIGNALISIEREN EINES ZUSTANDS EINES WACHSTUMSSUBSTRATS FÜR EINE PFLANZE**
METHOD AND CIRCUIT ARRANGEMENT FOR SIGNALING THE STATE OF A GROWTH SUBSTRATE FOR A PLANT
PROCÉDÉ ET CIRCUIT POUR SIGNALER UN ÉTAT D'UN SUBSTRAT DE CROISSANCE POUR UNE PLANTE

(30) Priorität: 24.02.2016 DE 102016202824
(43) Veröffentlichungstag der Anmeldung: 02.01.2019
(73) Patentinhaber: Reime, Gerd, 77815 Bühl (DE)
(72) Erfinder: Reime, Gerd, 77815 Bühl (DE)
(74) Vertreter: RPK Patentanwälte Reinhardt, Pohlmann und Kaufmann Partnerschaft mbB
(86) Internationale Anmeldenummer: PCT/EP2017/054136
(87) Internationale Veröffentlichungsnummer: WO 2017/144571

(56) Entgegenhaltungen:
- WO-A1-2011/083424
- DE-A1-102014 100 193
- DE-U1-202005 021 093
- US-A- 5 214 353
- US-A1- 2010 026 324
- US-B1- 6 198 398

## Beschreibung

Die Erfindung betrifft ein Verfahren sowie eine Schaltungsanordnung zum Signalisieren eines Zustands eines Wachstumssubstrats für eine Pflanze.

### Stand der Technik

Das Signalisieren eines Zustandes eines Wachstumssubstrats für eine Pflanze kann beispielsweise auf optischem Weg erfolgen, wobei abhängig von dem Zustand des Substrats ein optisches Signal wiedergegeben werden kann. Insbesondere ist es bekannt, eine Lichtquelle je nach Zustand des Wachstumssubstrats einzuschalten oder auszuschalten. So kann durch Betrachten der eingeschalteten oder ausgeschalteten Lichtquelle ein Hinweis auf den Zustand des Wachstumssubstrates signalisiert werden. Bei dem Wachstumssubstrat der Pflanze kann es sich beispielsweise um eine Pflanzenerde, ein Granulat oder ein anderes, für ein Wachstum von Pflanzen geeignetes Material handeln.

Aus der dem Oberbegriff der unabhängigen Ansprüche zu Grunde liegenden US 2010/026324 A1 sind ein Verfahren und eine Schaltungsanordnung zum Signalisieren eines Zustands eines Wachstumssubstrats bekannt.

Die DE 10 2014 100 193 A1 zeigt eine Vorrichtung sowie das zugehörige Verfahren zum Signalisieren eines Wachstumssubstrats für eine Pflanze, wobei Mittel zum Messen unter Verwendung einer Fotozelle vorgesehen sind. Mit Mitteln zur dauerhaften Wiedergabe von optisch wahrnehmbaren Blitzsignalen in Form von Leuchtelektroden wird der Zustand eines Wachstumssubstrats dem Benutzer signalisiert.

Aus der WO 2011/083424 A1 ist eine Schaltungsanordnung mit einer Photozelle als Energiequelle bekannt, bei der eine LED anhand eines spannungsbegrenzten Pulses versorgt wird. Die Spannungsbegrenzung erfolgt mittels einer separaten Diode. Aus der US 5,214,353 A ist ferner eine Schaltungsanordnung bekannt, bei der eine LED sowohl die Aufgabe der Spannungsbegrenzung als auch der optischen Anzeige übernimmt. Beide Dokumente betreffen jedoch ein vom vorliegenden technischen Gebiet des Signalisierens und Messens eines Zustands eines Wachstumssubstrates für eine Pflanze entferntes technisches Gebiet.

Aus der US 2009/0140865 A1 ist eine Vorrichtung zum kapazitiven Messen des Feuchtegehalts eines Wachstumssubstrats mit einer mehrfarbigen LED zur Anzeige des Zustands des Feuchtegehalts bekannt. Die Vorrichtung wird über eine externe Energieversorgung oder über eine Batterie mit Strom versorgt. Um Energie zu sparen kann die LED so angesteuert werden, dass sie nur ab einem bestimmten Trockenheitsgrad den Zustand anzeigt. Eine vergleichbare von einer Batterie versorgte Lösung ist aus der US 2010/0251807 A1 bekannt.

Die DE 37 02 725 A1 zeigt einen kapazitiven Feuchtesensor mit einem Kunststoffröhrchen, in dem ein ringförmiger Kondensator als Sensor eingegossen ist, zur Verwendung in automatischen Bewässerungsanlagen, wobei der Feuchtesensor über ein Netzgerät am Stromnetz anschließbar ist.

Aus der US 5,634,342 ist eine Lösung bekannt, die den Peltier-Effekt nutzt, um mittels des so gewonnenen Kondenswassers Heimpflanzen zu bewässern. Eine Energiequelle ist stets vorhanden und an eine Batterie oder Stromversorgung anzuschließen.

Aus der US 2014/0109658 A1 ist eine Messvorrichtung bekannt, die mit zwei Messfühlern an einem Baum angesetzt werden kann, um den Feuchtegehalt im Innern des Baums zu messen. Sie weist eine Anzeigevorrichtung auf und wird über eine Photozelle versorgt.

Es ist bekannt, optische Lichtaustrittsflächen mit dem Wachstumsgranulat in einen Anlagekontakt zu bringen und über eine messtechnische optische Auswertung Rückschlüsse auf den Zustand des Wachstumssubstrats zu erzielen. Aus der DE 10 2013 012 599 A1 ist ein optoelektronischer Sensor zur Erfassung der Bodenfeuchtigkeit mit wenigstens einer Lichtquelle bekannt, die Licht in einen Lichtleiter einkoppelt, und wenigstens einem Empfänger, der von der Lichtquelle beabstandet das im Lichtleiter befindliche, von der Lichtquelle stammende Licht empfängt und in ein elektrisches Empfangssignal umsetzt, sowie mit einer Auswerteeinheit zur Auswertung von Änderungen des elektrischen Empfangssignals zur Bestimmung der Bodenfeuchtigkeit, wobei der Lichtleiter so im Boden angeordnet ist, dass Licht in Abhängigkeit der Bodenfeuchtigkeit in den Boden transmittiert, soweit es nicht an der Wandung des Lichtleiters infolge von Bodenfeuchtigkeit aus dem Lichtleiter ausgekoppelt ist.

### Darstellung der Erfindung

Aufgabe der Erfindung ist es, ein möglichst gut wahrnehmbares und energiesparendes Signalisieren eines Zustandes eines Wachstumssubstrats für eine Pflanze zu ermöglichen.

Diese Aufgabe wird durch ein Verfahren mit den Merkmalen des Anspruches 1 und eine Schaltungsanordnung mit den Merkmalen des Anspruches 5 zum Signalisieren eines Zustands eines Wachstumssubstrats für eine Pflanze.

Vorteilhafte Weiterbildungen sind Gegenstand der abhängigen Patentansprüche.

Das Verfahren zum Signalisieren des Zustands des Wachstumssubstrats für die Pflanze weist die Merkmale des Anspruchs 1 auf.

Unter optisch unterscheidbar kann verstanden werden, dass sich ein Ort an einer entsprechenden Vorrichtung ändert, an dem das Blitzsignal signalisiert wird, sich eine Art des Signals ändert, insbesondere hinsichtlich Farbe, Länge, Intensität, Leuchtdauer, Pause zwischen einzelnen Blitzen und/oder Ähnliches.

Bei der physikalischen Größe kann es sich um eine das Wachstum der Pflanze positiv oder negativ beeinflussende Größe handeln, insbesondere einen Wassergehalt und/oder eine Feuchte des Wachstumssubstrats. Es sind jedoch auch andere Größen denkbar, beispielsweise ein Gehalt an Nährstoffen wie Dünger oder Ähnlichem. Vorteilhaft kann mittels der Blitzsignale symbolisiert werden, ob der Zustand des Wachstumssubstrats für ein Wachstum der Pflanze geeignet ist oder nicht, beispielsweise ausreichend Feuchtigkeit für das Wachstum enthält. Unter einem Blitzsignal kann ein zyklisches Aufblitzen eines Leuchtmittels verstanden werden. Insbesondere ist es denkbar, zum Übermitteln weiterer Informationen eine Leuchtdauer der einzelnen Blitze und/oder einen Zeitabstand der einzelnen Blitze und/oder eine Helligkeit der einzelnen Blitze zu steuern. Ferner ist es denkbar, mittels einer Farbe des Blitzsignales weitere Informationen wiederzugeben.

Insbesondere ist das Blitzsignal so gestaltet, dass eine einzelne Leuchtdauer eines einzelnen Blitzes sehr viel kürzer ist als eine Pause zwischen den einzelnen Blitzsignalen. Vorteilhaft kann durch das Blitzsignal, das insbesondere kurzzeitig eine vergleichsweise hohe Energiedichte aufweist eine besonders gute optische Wahrnehmung und damit Signalisierung bei vergleichsweise geringer Leuchtstärke des einzelnen Blitzes erzielt werden. Außerdem kann durch die vergleichsweise langen Pausen eine sehr energiesparende Signalisierung realisiert werden. Es ist denkbar, mittels des Verfahrens eine sogenannte Man-in-the-Loop-Regelung zu realisieren, insbesondere zum Regeln einer Bodenfeuchte des Wachstumssubstrats, vorliegend insbesondere als Zweipunktregler.

Zum Durchführen des Verfahrens ist es außerdem vorteilhaft möglich, ein Messen einer bei einer Pflanze und/oder dem Wachstumssubstrat herrschenden Helligkeit und ein Steuern zumindest einer der folgenden Eigenschaften des Blitzsignals in Abhängigkeit der gemessenen Helligkeit durchzuführen, insbesondere in Form einer Ausgangsspannung der Photozelle: Eine Leuchtdauer eines einzelnen Blitzes, eine Helligkeit, ein optisches Ausklingen oder Anschwellen eines einzelnen Blitzes, eine Dauer eines einzelnen Blitzes, ein zeitlicher Abstand zwischen zwei aufeinanderfolgenden Blitzen. Vorteilhaft kann eines der wenigstens zwei Blitzsignale derartig angesteuert werden. Insbesondere ist es denkbar, dies bei einer geringen Heiligkeit, insbesondere nachts vorzusehen. Vorteilhaft kann dadurch eine möglicherweise als störend empfundene besonders helle Signalisierung des Zustandes vermieden werden. Insbesondere ist es denkbar, nur eines der Blitzsignale derartig an die Helligkeit anzupassen, insbesondere dasjenige Blitzsignal, das einen wunschgemäßen Zustand des Wachstumssubstrats signalisiert. Insbesondere kann es sich dabei um eine grüne, also positive Signalisierung handeln. Die entsprechend gegenteilige Signalisierung, insbesondere durchgeführt mittels einer roten Farbe, die beispielsweise ein erforderliches Gießen der Pflanze signalisiert, kann vorteilhaft nicht in einer Leuchtstärke reduziert werden, sodass dies auch bei Nacht klar und deutlich wahrnehmbar ist, sodass gegebenenfalls die Pflanze gegossen wird. Außerdem ist es möglich, dadurch zusätzlich Energie einzusparen, wobei bei geringer Umgebungshelligkeit auch eine geringere Leuchtstärke zum Signalisieren ausreicht. Ferner ist es denkbar, durch die Anpassung des Blitzsignals an die Helligkeit eine vom Auge aufgrund seiner Adaptionsfähigkeit nur sehr schwer wahrnehmbare Änderung der Umgebungshelligkeit zu signalisieren, beispielsweise durch einen Abstand der einzelnen Blitze, sodass beispielsweise Informationen über eine Eignung eines Aufstellungsortes des die Pflanze aufweisenden Wachstumssubstrates zusätzlich möglich sind.

Vorteilhaft kann die vom Zustand des Wachstumssubstrats abhängige physikalische Größe durch Bestimmung der Begrenzung der Spannung eines das Blitzsignal hervorrufenden Pulses durch eine das Blitzsignal erzeugende LED gemessen werden. Dadurch kann stromsparend eine Zustandserkennung durch Auswertung des Pulses erfolgen, der gleichzeitig zur Ansteuerung der LED genutzt wird.

Vorzugsweise wird die von wenigstens einer Photozelle erzeugte Energie auf einem Energiespeicher wie einem Kondensator gespeichert, so dass ein konstanter Rückschlagimpuls zu gleicher Leuchtkraft und gleichen Messbedingungen beiträgt im Gegensatz zu einer sich allmählich entleerenden Batterie. Dabei ist die Spannung des Energiespeichers kleiner gleich der Versorgungsspannung der Anzeigevorrichtung.

Die Schaltungsanordnung zum Signalisieren des Zustands des Wachstumssubstrats weist die Merkmale des Anspruchs 5 auf.

In einer bevorzugten Ausführungsform muss die optische Anzeigevorrichtung zum Signalisieren des Zustandes nicht in einen Dauerbetrieb eingeschaltet werden. Vorteilhaft kann diese lediglich zum Wiedergeben eines kurzen Blitzes der Blitzsignale eingeschaltet werden. Insbesondere erfolgt das kurzzeitige Einschalten in Abhängigkeit von einer elektrischen Ausgangsgröße des Messfilters und/oder einer Dauer des spannungsbegrenzten Pulses. Bevorzugt kann das kurzzeitige Einschalten zyklisch wiederholt werden, sodass dennoch eine Beobachtung und damit Wahrnehmung des Blitzsignales zu einem beliebigen Zeitpunkt möglich ist. Alternativ oder zusätzlich ist es denkbar, mittels einer Benutzereingabe zunächst eine Messung und darauf basierend eine Signalisierung, insbesondere einen von der Messung abhängigen einzelnen Blitz zur Signalisierung auszulösen. Unter einem Blitzsignal kann ein optisches Anschwellen und Abschwellen verstanden werden, wobei eine Ausschaltdauer der entsprechenden Lichtquelle deutlich größer ist als eine Einschaltdauer. Insbesondere kann unter einem Blitzsignal ein kurzzeitiges Aufleuchten, insbesondere mit einer besonders hohen Flankensteilheit einer Lichtquelle verstanden werden. Gemäß der Erfindung ist der Puls spannungsbegrenzt, sodass das Messfilter während einer Messung stets mit einer konstanten Spannung versorgt werden kann. Dadurch kann eine möglichst genaue Messung zum Ermitteln des Zustandes und anschließenden Signalisieren erfolgen. Ferner wird das Messfilter vorteilhaft lediglich mit dem spannungsbegrenzten Puls elektrisch versorgt. Dies hat den zusätzlichen Vorteil, dass nicht nur die optische Anzeigevorrichtung, sondern auch das Messfilter besonders energiesparend betrieben werden kann. Vorteilhaft erfolgt also das aktive Signalisieren in Form des Aufblitzens und das Messen mittels des Messfilters maximal für eine Dauer des spannungsbegrenzten Pulses. Zwischen den einzelnen spannungsbegrenzten Pulsen ist es möglich, dass keine oder zumindest nur eine äußerst geringe Energiemenge zum Betreiben der Schaltungsanordnung verbraucht bzw. benötigt wird. Insofern kann unter kurzzeitig einschaltbar verstanden werden, dass eine Einschaltdauer der optischen Anzeigevorrichtung durch eine Länge des spannungsbegrenzten Pulses begrenzbar ist und/oder begrenzt ist. Damit ergibt sich also keine Notwendigkeit, das Messfilter und im Falle der optischen Anzeige auch die Anzeigevorrichtung mit einem Dauerstrom zu bestromen. Vorzugsweise erfolgt also lediglich eine kurzzeitige Messung und abhängig vom Zustand die entsprechende kurzzeitige Signalisierung. Es ist denkbar, falls der Zustand des Wachstumssubstrates für ein gutes Wachstum der Pflanze nicht in Ordnung ist, die optische Anzeigevorrichtung nicht kurzzeitig einzuschalten und, falls der Zustand in Ordnung ist, diese kurzzeitig und zyklisch wiederholt einzuschalten. Das kurzzeitige Einschalten kann also für eine zeitlich ausgedehnte Signalisierung zyklisch wiederholt erfolgen. Vorteilhaft kann zur Messung der Puls gefiltert und ein Übertragungsverhalten des Messfilters ausgewertet werden.

Nach einem bevorzugten Ausführungsbeispiel der Schaltungsanordnung weisen die elektrische Energiequelle und die optische Anzeigevorrichtung eine gemeinsame Diodenvorrichtung auf, mittels der gleichzeitig der Puls spannungsbegrenzbar und das Blitzsignal wiedergebbar sind. Vorteilhaft übernimmt die Diodenvorrichtung gleichzeitig die Wiedergabe des Blitzsignals und die Begrenzung des spannungsbegrenzten Pulses. Für die Spannungsbegrenzung gegebenenfalls benötigte Energie wird vorzugsweise entsprechend eines Wirkungsgrades der Diodenvorrichtung gleich in Licht zur Wiedergabe des Blitzsignals umgewandelt. Vorteilhaft sind dadurch eine besonders energiesparende Signalisierung des Zustandes des Wachstumssubstrates und gleichzeitig durch die Spannungsbegrenzung eine genaue Messung möglich.

Außerdem weist bevorzugt die elektrische Energiequelle einen der oder den Photozellen parallel geschalteten Energiespeicher zum Zwischenspeichern der elektrischen Energie auf. Für Dunkelphasen kann vorzugsweise diese photovoltaisch gewonnene Energie in dem parallel geschalteten Energiespeicher zwischengespeichert werden. Damit ist es vorteilhaft möglich, die Schaltungsanordnung auch bei geringem Lichteinfall weiterzubetreiben. Bei dem elektrischen Energiespeicher kann es sich um einen Akkumulator, einen Kondensator oder einen beliebigen anderen elektrischen Energiespeicher handeln. Ferner ist es optional denkbar, mittels der Photozelle eine auf die Pflanze einfallende Lichtmenge zu ermitteln, wobei insbesondere die so ermittelte Helligkeit mittels der Anzeigevorrichtung signalisierbar ist.

Ferner ist bevorzugt vorgesehen, dass die die elektrische Energiequelle eine Induktivität als weiteren elektrischen Energiespeicher aufweist, mittels der zum Erzeugen des spannungsbegrenzten Pulses ein Rückschlagimpuls erzeugbar ist. In diesem Fall weist die Energiequelle also einen weiteren elektrischen Energiespeicher, nämlich die Induktivität auf. In der Induktivität speicherbare elektrische Energie ist mittels eines Rückschlagimpulses entnehmbar. Dieser mittels der Induktivität erzeugbare Rückschlagimpuls kann vorteilhaft in einer Maximalspannung zum Erzeugen des spannungsbegrenzten Pulses begrenzt werden. Dies ist mittels der Diodenvorrichtung möglich. Insbesondere ist es denkbar, dass die Induktivität mittels eines elektrischen Schaltelements, insbesondere einem Transistor und/oder einem Feldeffekttransistor, für eine kurze Dauer bestrombar, also an die Photozelle und/oder den elektrischen Energiespeicher schaltbar ist, wobei die Induktivität der Diodenvorrichtung, insbesondere einer Leuchtdiode, vorgeschaltet ist. Mittels der Leuchtdiode kann die Spannung des Rückschlagimpulses begrenzt werden und daraus gleichzeitig die Energie zum optischen Signalisieren des Blitzsignals entnommen werden. So kann durch das Erzeugen des Rückschlagim-pulses eine Spannungserhöhung erfolgen. Es ist insbesondere denkbar, für ein Betreiben einer Leuchtdiode zu niedrige Spannungen, insbesondere zwischen 0,6 und 0,9 V, insbesondere bevorzugt zwischen 0,7 und 1,1 V, auf deutlich höhere Werte, insbesondere größer 3 V, insbesondere zwischen 20 und 60 V zu erhöhen. Dies kann vorzugweise während einer schlechten Beleuchtung der Photozelle erfolgen, wobei diese nur vergleichsweise geringe Spannungen liefert. Außerdem können durch die Spannungsbegrenzung des Rückschlagimpulses auch deutlich höhere Versorgungsspannungen der Photozelle verarbeitet werden, ohne dadurch die Versorgung der übrigen Schaltungsanordnung mit elektrischer Energie bzw. Spannung wesentlich zu verändern. Die elektrische Energiequelle kann also vorteilhaft insgesamt drei Arten von Teilenergiequellen aufweisen, nämlich die Photozelle, den elektrischen Energiespeicher, insbesondere Kondensator, und die Induktivität.

Eine vorteilhafte Ausgestaltung der Schaltungsanordnung sieht vor, dass die elektrische Energiequelle eine Pulssteuerung aufweist, mittels der die Induktivität zum Erzeugen des Rückschlagimpulses kurzzeitig bestrombar ist. Unter einer Pulssteuerung kann insbesondere ein Pulsgenerator mit einem nachgeschalteten Schaltelement zum Bestromen der Induktivität während einer Pulsdauer verstanden werden. Vorteilhaft sind Pulsgeneratoren mit einer äußerst geringen Stromaufnahme bekannt, insbesondere kleiner als 1 µA, vorzugsweise kleiner als 500 nA, bevorzugt kleiner als 300 nA, sodass vorteilhaft zum Messen und Signalisieren ein Durchschnittsstrom von lediglich 500 nA bei einer Spannung von 0,6 bis 3 V, insbesondere 0,7 bis 2,5 V, möglich ist. Dies ermöglicht einen besonders stromsparenden Nachtbetrieb.

Bei einem weiteren bevorzugten Ausführungsbeispiel der Schaltungsanordnung ist vorgesehen, dass das Messfilter und die optische Anzeigevorrichtung in der Zeit, in der kein spannungsbegrenzter Puls vorliegt, im Wesentlichen stromlos sind. Vorteilhaft verbrauchen die zum eigentlichen Messen und Anzeigen notwendigen Bauelemente der Schaltungsanordnung zwischen den Pulsen keine elektrische Energie. Unter im Wesentlichen stromlos kann verstanden werden, dass zwischen den Pulsen kein Strom oder zumindest nur ein äußerst geringer Strom fließt, der insbesondere den sogenannten Leckageströmen entspricht.

Bei einer weiteren Schaltungsanordnung ist es vorteilhaft denkbar, dass die optische Anzeigevorrichtung einen durch den spannungsbegrenzten Puls mit elektrischer Energie versorgbaren Vierpol aufweist, der in einem ersten Parallelzweig eine erste Leuchtdiode der Diodenvorrichtung sowie ein erstes elektrisches Schaltelement zum Schalten der ersten Leuchtdiode und in einem zweiten Parallelzweig eine zweite Leuchtdiode der Diodenvorrichtung sowie ein zweites elektrisches Schaltelement zum Schalten der zweiten Leuchtdiode aufweist, wobei ein erster Schalteingang des ersten elektrischen Schaltelements der zweiten Leuchtdiode nachgeschaltet ist und ein zweiter Schalteingang des zweiten elektrischen Schaltelements einem Messausgang des Messfilters nachgeschaltet ist.

Vorteilhaft kann die zweite Leuchtdiode mittels des zweiten Schaltelements abhängig von einer Ausgangsspannung des Messausgangs des Messfilters geschaltet werden, insbesondere in Form des Blitzsignales maximal für die Dauer des spannungsbegrenzten Pulses kurzzeitig eingeschaltet werden. Vorzugsweise kann bei einer bestimmten Ausgangsspannung des Messausgangs das erste Schaltelement durchgeschaltet werden, während das zweite Schaltelement sperrt. Dadurch ist es möglich, zwei unterschiedliche optische Signale wiederzugeben, beispielsweise für den Zustand ,in Ordnung' und ,nicht in Ordnung' des Wachstumssubstrates, insbesondere durch unterschiedliche Farben, insbesondere durch eine grüne und rote Wiedergabe des Blitzsignals. Außerdem kann das Messfilter eine über der Zeit veränderliche Ausgangsspannung erzeugen, die insbesondere während der Dauer des spannungsbegrenzten Pulses ansteigt. In diesem Fall ist es denkbar, dass die zwei elektrischen Schaltelemente nacheinander durchschalten, sodass für einen Betrachter der optischen Anzeigevorrichtung sich eine gleichzeitige Signalisierung des Zustandes ,in Ordnung' und ,nicht in Ordnung' ergibt, da auch das zeitliche Verhalten des Messausgangs des Messfilters eine Information über den Zustand des Wachstumssubstrates enthält, kann dadurch vorteilhaft ein dritter Zustand des Wachstumssubstrates signalisiert werden, beispielsweise ein für das Wachstum der Pflanze optimaler Zustand des Wachstumssubstrats. Vorteilhaft kann ein mittels des Messausgangs ansteuerbarer Schwellwertschalter realisiert werden.

Bei dem Messfilter kann es sich insbesondere um einen Hochpassfilter oder Tiefpassfilter handeln. Durch die kurzzeitige während der Dauer des spannungsbegrenzten Pulses erfolgende Bestromung des Messfilters ergibt sich eine über der Zeit veränderliche elektrische Ausgangsgröße, deren zeitlicher Verlauf von der Kapazität und dadurch von dem Zustand des Wachstumssubstrates abhängt. Relevant kann insbesondere sein, ob innerhalb der Pulsdauer eine Schaltspannung des Schwellwertschalters erreicht wird oder nicht. Bei dem Zustand des Wachstumssubstrates kann es sich um einen Wert einer Bodenfeuchte handeln. Das Wachstumssubstrat dient als Dielektrikum der Kapazität , sodass sich also abhängig von einem Feuchtegrad unterschiedliche Werte der Kapazität ergeben. Dazu wird die Kapazität mit dem Wachstumssubstrat in eine Wechselwirkung gebracht, insbesondere wird sie in dieses eingesteckt.

Die Kapazität weist erste Elektrode und eine zweite Elektrode auf, die insbesondere mit einem Schutzlack versehen sind.

Die Elektroden der Kapazität können vorteilhaft mit dem Wachstumssubstrat in einen Anlagekontakt gebracht werden. Damit kann dadurch das Wachstumssubstrat als Dielektrikum wirken. Da Wasser eine äußerst gute Permittivität aufweist, also ein besonders gutes Dielektrikum ist, kann auf diese Art und Weise eine besonders genaue Messung des Zustandes des Wachstumssubstrates erfolgen. Vorteilhaft ist es insbesondere denkbar, dass die Elektroden der Kapazität auf eine einfache Platine, wie sie zum Herstellen von elektrischen Schaltkreisen üblicherweise verwendet wird, aufgebracht sind, insbesondere als Kupferbeschichtung. Die so erzeugbaren Elektroden können auf einfache Art und Weise durch ein Einstecken der Platine samt den aufgedruckten Elektroden in das Wachstumssubstrat mit diesem in eine Wirkverbindung gebracht werden. Die Elektroden sind voneinander beabstandet auf der Platine angebracht. Besonders vorteilhaft ist es, wenn die Elektroden auf gegenüberliegenden Seiten der Platine angebracht sind. Dadurch ergibt sich ein vergleichsweise langer Weg zwischen den Elektroden, wodurch vorteilhaft ein möglicherweise stattfindender lonentransport reduziert werden kann, wodurch eine möglichst lange Lebensdauer der Elektroden und dadurch eine langzeitstabile besonders genaue Messung des Zustandes möglich sind. Außerdem können die Elektroden durch den Schutzlack zusätzlich geschützt sein, was deren Lebensdauer außerdem erhöht. Insbesondere ist es denkbar, als Schutzlack einen üblicherweise bei elektrischen Schaltkreisen mit Platinen verwendeten Lötstopplack zu verwenden. Schließlich sind besonders vorteilhaft die zwei Elektroden zwischen jeweiligen spannungsbegrenzten Pulsen stromlos, sodass in dieser Zeit ebenfalls kein lonentransport zwischen den Elektroden erfolgt. Über die Elektroden wird also vorzugsweise kein Gleichstrom geleitet, wobei vorteilhaft ein niedriger Energieverbrauch und eine große Standzeit der Elektroden erzielt werden können.

Schließlich ist es bei einer vorteilhaften Ausgestaltung der Schaltungsanordnung möglich, dass der Kapazität ein elektrischer Widerstand und/oder ein einstellbarer elektrischer Widerstand vorgeschaltet ist/sind.

Mittels des Widerstandes kann eine Empfindlichkeit des Messfilters, insbesondere Hochpass-/Tiefpassfilters, eingestellt werden. Vorteilhaft ist damit zusammen mit einer Pulsdauer des spannungsbegrenzten Pulses eine Schwelle vorgebbar, ab der der entsprechende Zustand, also in Ordnung oder nicht in Ordnung des Wachstumssubstrates signalisierbar ist. Außerdem ist es denkbar, eine weitere Schwelle vorzugeben, wobei zwischen den beiden Schwellen beide Zustände gleichzeitig signalisiert werden, was wie vorab beschrieben als insbesondere optimaler Zustand des Wachstumssubstrats interpretierbar ist. Diese weitere Schwelle kann insbesondere durch Auslegung eines Innenwiderstands der Induktivität sowie einer Größe derselben vorgegeben werden, die eine Länge des spannungsbegrenzten Pulses beeinflussen. Zusammen mit dem zeitlichen Verhalten des Ausgangs des Messfilters, dem Vierpol und der Dauer des spannungsbegrenzten Pulses ist vorteilhaft die Anzeige von drei Zuständen des Wachstumssubstrates auf eine besonders energiesparende und genau messende Art und Weise möglich.

Die Messvorrichtung weist eine vorab beschriebene Schaltungsanordnung auf. Es ergeben sich die vorab beschriebenen Vorteile. Die Photozelle der Messvorrichtung kann eine wahrnehmbare oder ablesbare Botschaft aufweisen. Die Leuchtdioden sind bevorzugt benachbart zu der Photozelle und/oder auf gegenüberliegenden Seiten der Photozelle angeordnet. Dies bewirkt eine gute Ablesbarkeit der Botschaft, insbesondere beiläufig wenn ein Betrachter einen Messwert der Messvorrichtung an den Leuchtdioden abliest.

Weitere Vorteile, Merkmale und Einzelheiten ergeben sich aus der nachfolgenden Beschreibung, in der - gegebenenfalls unter Bezug auf die Zeichnung - zumindest ein Ausführungsbeispiel im Einzelnen beschrieben ist.

Gleiche, ähnliche und/oder funktionsgleiche Teile sind mit gleichen Bezugszeichen versehen.

### Kurzbeschreibung der Figuren

Im Folgenden wird die Erfindung an Hand von in den Figuren dargestellten Ausführungsbeispielen näher erläutert. Es zeigen:
- Fig. 1A: eine Seitenansicht einer Pflanzanordnung mit einer in einem Wachstumssubstrat eingepflanzten Pflanze und einer einen Zustand des Wachstumssubstrats überwachenden Messvorrichtung;
- Fig. 1B: einen Querschnitt entlang der in Fig. 1A gezeigten Linie I-I durch zwei Messelektroden der Messvorrichtung;
- Fig. 2: ein Blockschaltbild der in Fig. 1A gezeigten Messvorrichtung;
- Fig. 3A: einen Schaltplan der in Fig. 1A gezeigten Messvorrichtung;
- Fig. 3B: eine Detailansicht eines in Figur 3 A gezeigten Messwiderstands als veränderlicher Widerstand; und
- Fig. 4: ein Ablaufdiagramm eines insbesondere mittels der in Fig. 1A gezeigten Messvorrichtung durchführbaren Verfahrens zum Signalisieren eines Zustands der Pflanzanordnung.

Ausführliche Beschreibung bevorzugter Ausführungsbeispiele Die Erfindung wird jetzt beispielhaft unter Bezug auf die beigefügten Zeichnungen näher erläutert. Allerdings handelt es sich bei den Ausführungsbeispielen nur um Beispiele.

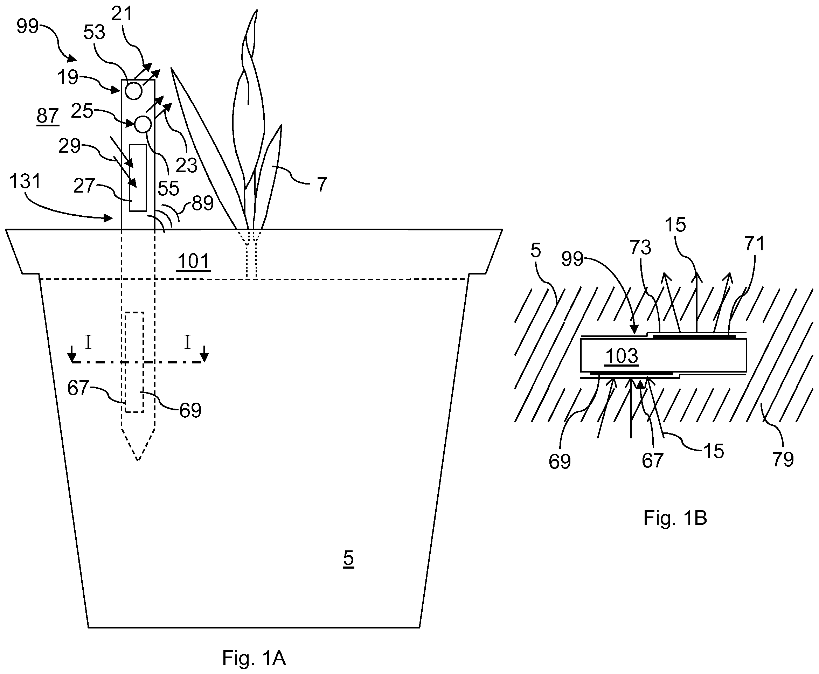

Fig. 1 zeigt eine Pflanzanordnung 101 mit einem Wachstumssubstrat 5, einer in dem Wachstumssubstrat 5 eingepflanzten Pflanze 7 sowie einer Messvorrichtung 99 zum Überwachen und Signalisieren eines Zustandes 3 des Wachstumssubstrats 5. Die Messvorrichtung 99 ist insbesondere zum Messen und Signalisieren eines Feuchtigkeitsgehalts des Wachstumssubstrats 5 ausgelegt.

Die Messvorrichtung 99 weist zumindest eine erste Lichtquelle auf, die eine erste Leuchtdiode 53 umfasst, und vorzugsweise eine zweite Lichtquelle, die eine zweite Leuchtdiode 55 umfasst. Die erste Leuchtdiode 53 ist insbesondere grün und die zweite Leuchtdiode 55 rot. Die Leuchtdioden 53 und 55 sind beabstandet zueinander angeordnet und dienen zum Signalisieren des Zustandes 3 des Wachstumssubstrats 5. Mittels den Leuchtdioden 53 und 55 kann ein erstes Blitzsignal 21 und ein zweites Blitzsignal 23 wiedergegeben werden, das in Fig. 1A jeweils mit zwei kleinen parallelen Pfeilen symbolisiert ist. Das erste Blitzsignal 21, das insbesondere in grüner Farbe aussendbar ist, kann einen ausreichenden Wassergehalt des Wachstumssubstrats 5 signalisieren. Das zweite Blitzsignal 23 kann einen nicht ausreichenden Wassergehalt des Wachstumssubstrats 5 signalisieren. Bevorzugt ist es auch möglich, dass ein Wiedergeben beider Blitzsignale 21 und 23 einen weiteren Zustand des Wachstumssubstrats 5, beispielsweise einen für das Wachstum der Pflanze 7 optimalen Wassergehalt signalisieren. Dabei ist es denkbar, dass die Blitzsignale 21 und 23 synchron, also jeweils gleichzeitig wiedergegeben werden, oder asynchron, bevorzugt so kurz nacheinander, dass dies als eine einheitliche Signalisierung wahrnehmbar ist.

Die Messvorrichtung 99 ist mittels einer Photozelle 27 mit elektrischer Energie versorgbar. Bevorzugt kann die Photozelle 27 als Wiedergabefläche für ein Logo und/oder einen Schriftzug verwendet werden. Um eine gute Wahrnehmbarkeit des Logos und/oder des Schriftzugs zu ermöglichen, ist die Photozelle 27 bevorzugt benachbart zu oder zwischen den Leuchtdioden 53 und 55 angeordnet. Die Photozelle 27 dient zum Umwandeln von Licht 29 in elektrische Energie. Außerdem ist es bevorzugt möglich, mittels der Photozelle 27 eine Umgebungshelligkeit 87, also eine Intensität des auf die Photozelle 27 auftreffenden Lichtes 29 zu messen. Zusätzlich ist es möglich, den Zustand des Wachstumssubstrats 5 auch mittels eines akustisch wahrnehmbaren Signales 89 zu signalisieren. Außerdem weist die Photozelle 27 bevorzugt eine Oberfläche auf, mittels der eine Botschaft, insbesondere aufgedruckt, signalisiert werden kann. Insbesondere sind dazu die Leuchtdioden 53 und 55 benachbart zu der Oberfläche der Photozelle 27 angeordnet, um eine bessere Wahrnehmbarkeit der Botschaft beim Ablesen und/oder Wahrnehmen der Leuchtdioden 53 und 55 zu bewirken. Insbesondere kann so ein gewolltes zufälliges Wahrnehmen der Botschaft gefördert werden. Insbesondere können die Leuchtdioden 53 und 55 oberhalb und unterhalb beziehungsweise auf gegenüberliegenden Seiten der Oberfläche der Photozelle 27 angeordnet werden, sodass die Botschaft beim Ablesen derselben zwangsläufig in einem Blickfeld eines Betrachters ist.

Fig. 1B zeigt einen Querschnitt entlang der in Fig. 1A gezeigten Linie I-I der Messvorrichtung 99. In den Fig. 1A und 1B ist zu erkennen, dass die Messvorrichtung 99 zumindest teilweise in das Wachstumssubstrat 5 eingebracht ist. Insbesondere sind eine erste Elektrode 69 sowie eine zweite Elektrode 71 der Messvorrichtung 99 in das Wachstumssubstrat 5 eingebracht. Die Elektroden 69 und 71 stehen miteinander und mit dem Wachstumssubstrat 5 in einer elektrischen Wechselwirkung 15. Genauer handelt es sich bei den Elektroden 69 und 71 um eine Kapazität 67 bzw. bilden diese einen Kondensator aus. Dabei stellt das Wachstumssubstrat 5 ein Dielektrikum der Kapazität 67 dar, wobei sich zwischen den Elektroden 69 und 71 aufbauende elektrische Feldlinien durch das Wachstumssubstrat 5 hindurch verlaufen. Vorteilhaft ändert sich dadurch ein Wert der Kapazität 67 abhängig von einem Wassergehalt des Wachstumssubstrats 5. Diese elektrische Wechselwirkung 15 kann zum Ermitteln des Zustandes 3 des Wachstumssubstrats 5 bzw. zum Ermitteln einer physikalischen Größe 79 des Wachstumssubstrates 5 vorteilhaft ausgenutzt werden. Die physikalischen Größe 79 kennzeichnet das Wachstumssubstrat 5.

Gemäß einer bevorzugten Alternative sind die Elektroden 69 und 71 der Kapazität 67 der Messvorrichtung 99 nicht in einem direkten Anlagekontakt mit dem Wachstumssubstrat 5, das in Fig. 1B schraffiert symbolisiert ist. Die Elektroden 69 und 71 stehen dazu über einen Schutzlack 73 in einem mittelbaren Anlagekontakt mit dem Wachstumssubstrat 5. Vorteilhaft kann dadurch ein unerwünschter lonentransport zwischen den Elektroden 69, 71 und dem Wachstumssubstrat 5 sicher vermieden werden.

Wie in Fig. 1B ersichtlich, sind die Elektroden 69 und 71 beabstandet zueinander angeordnet. Bevorzugt sind diese gegenüberliegend beabstandet auf einer Leiterplatte 103 angebracht. Bei dem Schutzlack 73 handelt es sich bevorzugt um einen zum Herstellen der Leiterplatte 103 ohnehin verwendeten Lötstopplack. Vorteilhaft sind also zur Herstellung der Leiterplatte 103 und zum Schützen der Elektroden 69 und 71 keine zusätzlichen Fertigungsschritte erforderlich. Vorteilhaft kann das Schützen der Elektroden gleichzeitig mit dem Aufbringen des Lötstopplackes in Funktion des Schutzlackes 73 erfolgen.

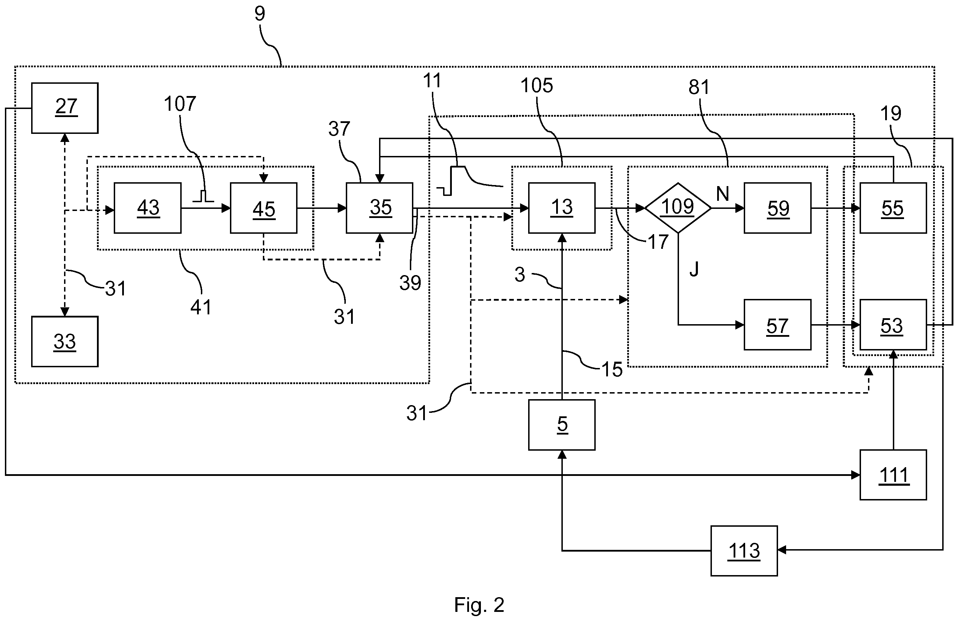

Fig. 2 zeigt ein Blockschaltbild der in den Fig. 1A und 1B gezeigten Messvorrichtung 99. Elektrische Energieflüsse sind gestrichelt und Systemgrenzen gepunktet dargestellt. Die Systemgrenzen dienen zur Erklärung der Funktionalität und/oder können je nach Auslegung der Messvorrichtung 99 und/oder oder gedanklichem Ansatz variieren.

Die Messvorrichtung 99 weist eine elektrische Energiequelle 9 auf, die eine Messschaltung 105, eine dieser nachgeschaltete Signalisierungssteuerung 81 sowie eine dieser nachgeschalteten optische Anzeigevorrichtung 19 mit elektrischer Energie 31 versorgt. Die elektrische Energiequelle 9 ist Teil einer Schaltungsanordnung 1 der in den Fig. 1A und 1B gezeigten Messvorrichtung 99. Die elektrische Energiequelle 9 weist die Photozelle 27 auf, mittels der aus dem einfallenden Licht 29 die elektrische Energie 31 gewinnbar ist. Diese kann vorteilhaft in einem elektrischen Energiespeicher 33, beispielsweise einem Kondensator und/oder einer Akkumulator zwischengespeichert werden. Der Fluss der elektrischen Energie 31, der in Fig. 2 gestrichelt dargestellt ist, kann also wahlweise von der Photozelle 27 oder von dem elektrischen Energiespeicher 33 ausgehen und dient zur Versorgung einer Pulssteuerung 41, die ebenfalls Teil der elektrischen Energiequelle 9 ist. Die Pulssteuerung 41 weist einen Pulsgenerator 43 auf. Mittels des Pulsgenerators 43 kann ein Rechteckimpuls erzeugt werden. Bei dem Pulsgenerator 43 kann es sich um einen handelsüblichen Pulsgenerator handeln, beispielsweise einen Pulsgenerator mit einem sehr geringen Energieverbrauch, insbesondere einem Energieverbrauch ≤ 300 nA bei insbesondere ca. 1 V Versorgungsspannung. Der Pulsgenerator 43 kann als Taktgeber verwendet werden, insbesondere so konfiguriert sein, dass dieser alle 2 - 5 s, insbesondere ca. alle 3 s, einen Taktpuls 107 liefert. Der Taktpuls kann eine Länge von ca. 200 µs, insbesondere 50 bis 550 µs, insbesondere kleiner 500 µs, aufweisen.

Außerdem weist die Pulssteuerung 41 ein elektrisches Pulsschaltelement 45 auf, insbesondere einen Transistor und/oder Feldeffekttransistor. Der Taktpuls 107 dient als Eingangsgröße des elektrischen Pulsschaltelements 45, wobei das elektrische Pulsschaltelement 45 den Fluss der elektrischen Energie 31 bei Vorhandensein des Taktpulses 107 durchschaltet.

Die elektrische Energiequelle 9 weist eine der Pulssteuerung 41 nachgeschaltete Induktivität 35 auf. Die Induktivität 35 wird mittels des elektrischen Pulsschaltelements 45 angesteuert. Genauer wird diese für die Dauer des Taktpulses 107 des Pulsgenerators 43 mittels des elektrischen Pulsschaltelements 45 an die Photozelle 27 und den elektrischen Energiespeicher 33 angeschaltet, also kurzzeitig von diesen bestromt. Dabei baut sich an der Induktivität 35 ein elektrisches Feld auf, in dem ein Teil der elektrischen Energie 31 speicherbar ist. Bei Abfallen des Taktpulses 107 schließt das elektrische Pulsschaltelement 45 der Pulssteuerung 41, wobei sich an der Induktivität 35 ein Rückschlagimpuls 39 aufbaut. Dieser Rückschlagimpuls 39 kann vorteilhaft mittels einer der Induktivität 35 nachgeschalteten Diodenvorrichtung 25 spannungsbegrenzt werden. Der Rückschlagimpuls 39 wird durch die Spannungsbegrenzung als Energiequelle zum Erzeugen eines spannungsbegrenzten Pulses 11 verwendet. Der spannungsbegrenzte Puls ist Teil des Flusses der elektrischen Energie 31 und dient zur Versorgung der nachgeschalteten Messschaltung 105, der Signalisierungssteuerung 81 sowie der optischen Anzeigevorrichtung 19 mit elektrischer Energie. Vorteilhaft sind diese nur während des Vorhandenseins des spannungsbegrenzten Pulses 11 bestromt. Vorteilhaft kann dadurch eine besonders energiesparende Schaltungsanordnung 1 realisiert werden. Zwischen den entsprechenden Pulsen 107 und 11 ist die Schaltungsanordnung 1 im Wesentlichen stromlos, wobei allenfalls geringfügige Leckageströme auftreten. Bei dem gezeigten Ausführungsbeispiel treten diese während der Taktpausen des Taktpulses 107 nur an dem elektrischen Pulsschaltelement 45 und während des Bestromens der Induktivität 35, also während der Dauer des Taktpulses 107, allenfalls in sehr geringem Umfang in der Signalisierungssteuerung 81 auf.

Der Rückschlagimpuls 39 der Induktivität 35 weist eine wesentlich höhere Spannung auf als die Photozelle 27 und/oder der elektrische Energiespeicher 33 liefern/t. Vorteilhaft kann dadurch eine Spannungsanhebung erfolgen, die besonders vorteilhaft mittels der Diodenvorrichtung 25 auf einen im Wesentlichen konstanten Wert heruntergeregelt werden kann. Unter im Wesentlichen konstant kann eine Regelabweichung von einem Konstantwert verstanden werden, die bei einem Proportionalregler und/oder Überproportionalregler, insbesondere mit einer vergleichsweise steilen Reglerkennlinie, insbesondere einer Regelung mittels einer Diode auftritt.

Zusammenfassend weist die elektrische Energiequelle 9 bevorzugt die Photozelle 27, den elektrischen Energiespeicher 33 zum Zwischenspeichern der von der Photozelle 27 gelieferten elektrischen Energie 31 sowie als weiteren elektrischen Energiespeicher 37 die Induktivität 35 auf. Außerdem weist die elektrische Energiequelle 9 eine Spannungsregelung zum Spannungsbegrenzen des Rückschlagimpulses 39 auf. Die Diodenvorrichtung 25 ist demnach auch Teil der elektrischen Energiequelle 9. Die elektrische Energiequelle 9 liefert vorteilhaft taktweise eine im Wesentlichen konstante Spannung, die vorteilhaft als Messspannung zur Versorgung des Messfilters 105 verwendbar ist. Die dazu benötigte elektrische Energie 31 ist durch Umwandlung von Licht 29 erzeugbar.

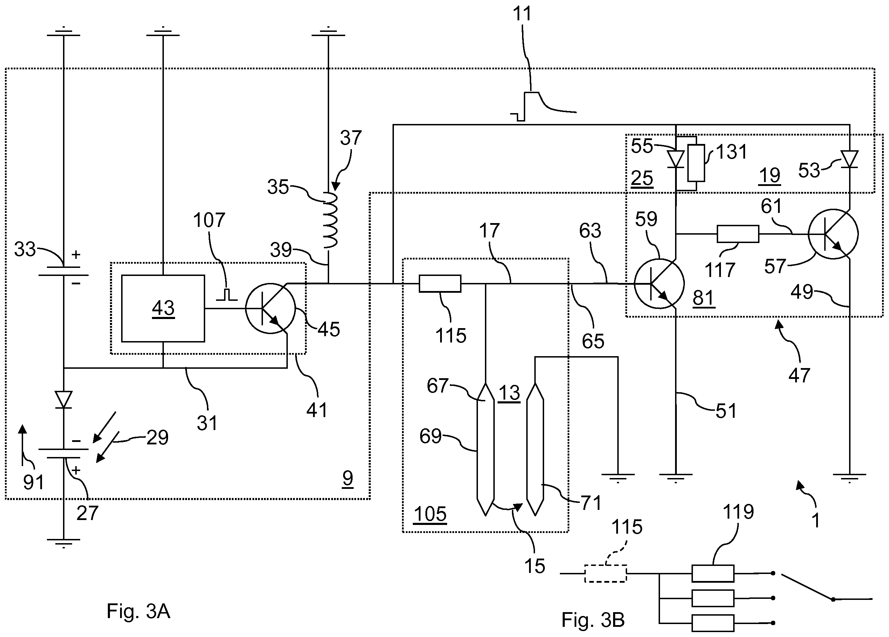

Die Messschaltung 105 weist ein Messfilter 13 auf. Das Messfilter 13 hat ein von dem Zustand 3 des Wachstumssubstrats 5 abhängiges Übertragungsverhalten. Dazu weist das Messfilter 13 die in den Fig. 1A und 1B gezeigten Elektroden 69 und 71 auf, die mit dem Wachstumssubstrat 5 in der elektrischen Wechselwirkung 15 stehen, sowie einen in Figur 3 gezeigten Messwiderstand 115 auf.

Das Messfilter 13 weist eine von der elektrischen Wechselwirkung 15, also dem Zustand 3 des Wachstumssubstrats 5 abhängige elektrische Ausgangsgröße 17 auf. Die elektrische Ausgangsgröße 17 dient zur Steuerung der Signalisierungssteuerung 81. Die Signalisierungssteuerung 81 weist zumindest eine Bedingung 109 auf, vergleicht also insbesondere die elektrische Ausgangsgröße 17 mit einem Schwellwert. Falls die Bedingung 109 erfüllt ist, wird ein erstes elektrisches Schaltelement 57 durchgeschaltet und die erste Leuchtdiode 53 bestromt. Dadurch kann der Zustand 3, genügend Feuchtigkeit, signalisiert werden. Die erste Leuchtdiode 53 wird mittels des ersten Schaltelements 57 gesteuert. Vorteilhaft dient die erste Leuchtdiode 53, während sie von dem spannungsbegrenzten Puls 11 bestromt wird, gleichzeitig als Spannungsbegrenzer des Rückschlagimpulses 39. Die erste Leuchtdiode 53 steuert also, während sie bestromt ist, die Induktivität 35, genauer deren Ausgangsspannung und damit zusammenhängend den an einem Innenwiderstand anfallenden Spannungsanteil des Rückschlagimpulses 39. Ein nicht erwünschter überschüssiger Spannungsanteil des Rückschlagimpulses 39 fällt dabei an einem Innenwiderstand der Induktivität 35 ab.

Falls die Bedingung 109 nicht erfüllt ist, wird ein zweites elektrisches Schaltelement 59 durchgeschaltet, sodass die zweite Leuchtdiode 55 von der Induktivität 35 mit der elektrischen Energie 31 versorgbar, also bestrombar ist. In diesem Fall wird also jeweils für die Dauer des spannungsbegrenzten Pulses 11 ein Blitz des zweiten Blitzsignals 23 wiedergegeben. Ebenso ist dies, falls das erste Schaltelement 57 die erste Leuchtdiode 53 ansteuert, zum Wiedergeben eines Blitzes des ersten Blitzsignals 21.

Auch die zweite Leuchtdiode 55 ist Teil der Spannungsregelung der elektrischen Energiequelle 9, steuert also die Induktivität 35 bzw. begrenzt den Rückschlagimpuls 39 auf den spannungsbegrenzten Puls 11. Es ist denkbar, eine der Leuchtdioden 53, 55 der Diodenvorrichtung 25 durch eine normale Diode zu ersetzen, sodass lediglich das Erfüllen oder nicht Erfüllen der Bedingung 109 aktiv optisch signalisierbar ist. Ferner ist es denkbar, für die Bedingung 109 zwei Schwellen vorzusehen, wobei beide Blitzsignale 21 und 23 wiedergebbar sind, solange sich die physikalische Größe 79, die durch die elektrische Ausgangsgröße 17 repräsentiert wird, zwischen den Schwellen befindet. Insbesondere kann die Bedingung 109 für unterschiedliche Parameter der elektrischen Ausgangsgröße 17 festgelegt werden, beispielsweise für eine Dauer der elektrischen Ausgangsgröße 17 sowie eine Spannung der elektrischen Ausgangsgröße 17. Insbesondere ist die elektrische Ausgangsgröße 17 durch das Übertragungsverhalten des Messfilters 13 über einer Zeit veränderlich. Insbesondere stellt die elektrische Ausgangsgröße 17 eine Antwort des Messfilters 13 auf eine Sprungfunktion dar. Insbesondere kann dazu der spannungsbegrenzte Puls 11 als Sprungfunktion verstanden werden. Vorteilhaft ist es also denkbar, während der Sprungantwort des Messfilters 13 als Bedingung 109 eine Schwelle für die Sprungantwort des Messfilters abzuprüfen, wobei es dadurch während der Dauer des spannungsbegrenzten Pulses 11 kurz nacheinander zum Aufleuchten beider Leuchtdioden 53 und 55 kommen kann. Vorteilhaft kann dies zum Signalisieren eines dritten Zustandes verwendet werden oder wie vorab beschrieben als Vorgabe einer weiteren Schwelle verstanden werden. Das Vorgeben der weiteren Schwelle bestünde dann in einer Länge des spannungsbegrenzten Pulses 11 bzw. in einer Auslegung der Induktivität 35, insbesondere hinsichtlich eines Innenwiderstandes, in Verbindung mit der Auslegung des Übertragungsverhaltens des Messfilters 13.

Gemäß einer vorteilhaften Alternative ist es möglich, zumindest eine der Leuchtdioden 53, 55, insbesondere die erste grüne Leuchtdiode 53 in einer Leuchtstärke zu steuern. Dies kann beispielsweise mittels eines Dimmers 111 erfolgen. Der Dimmer 111 kann dazu auf bekannte Art und Weise ein elektrisches Schaltelement wie einen Transistor, der bevorzugt durch eine Ausgangsspannung der Photozelle angesteuert ist, und/oder eine Pulssteuerung, eine Steuerung einer Pulslänge und/oder Ähnliches aufweisen. Der Dimmer 111 ist also in einer entsprechenden Steuerkette der Photozelle 27 nachgeschaltet und der ersten Leuchtdiode 53 vorgeschaltet. Vorteilhaft dient die Photozelle 27 dabei nicht nur zum Erzeugen der elektrischen Energie 31, sondern auch zur Messung der in der Umgebung der Messvorrichtung 99 herrschenden Helligkeit.

Mittels der in Fig. 2 als Blockschaltbild dargestellten Schaltungsanordnung 1 kann eine Regelung des Zustandes 3 des Wachstumssubstrats 5 mittels einer sogenannten Man-in-the-Loop-Regelung realisiert werden. Dazu kann von einer Person 113 die optische Anzeigevorrichtung 19 beobachtet und/oder abgelesen werden, beispielsweise einmal täglich. Je nach Signalisierung nimmt die Person 113 Einfluss auf den Zustand 3 des Wachstumssubstrats 5, beispielsweise durch Gießen. Wie vorab beschrieben, wirkt der Zustand 3 des Wachstumssubstrates 5 durch die Messung des Messfilters 13 auf die Schaltungsanordnung 1 zurück, sodass der Regelkreis zum Regeln des Zustandes 3 geschlossen ist.

Fig. 3 zeigt einen elektrischen Schaltplan der in Fig. 2 als Blockschaltbild beschriebenen Schaltungsanordnung 1. Zu erkennen sind die elektrische Energiequelle 9, die Messschaltung 105, die Signalisierungssteuerung 81 sowie die optische Anzeigevorrichtung 19. Die elektrische Energiequelle 9 weist den elektrischen Energiespeicher 33, insbesondere einen Kondensator, und diesem parallel geschaltet die Photozelle 27 auf. Außerdem ist der Photozelle 27 eine Schutzdiode in Reihe geschaltet, die den elektrischen Energiespeicher 33, insbesondere in Schwachlichtphasen, vor einer Entladung schützt. Die Photozelle 27 liefert bei einer Beleuchtung durch das Licht 29 eine elektrische Versorgungsspannung 91. Die entsprechende elektrische Energie 31 wird in dem elektrischen Energiespeicher 33 zwischengespeichert, sodass auch bei geringem Lichteinfall die elektrische Versorgungsspannung 91 zur Verfügung steht. Mit der elektrischen Versorgungsspannung 91 wird der an den elektrischen Energiespeicher 33 und die Photozelle 27 angeschlossene Pulsgenerator 43 elektrisch versorgt. Der Pulsgenerator 43 liefert an einem Ausgang den Taktpuls 107. Der Ausgang des Pulsgenerators 43 ist an die Basis des elektrischen Pulsschaltelements 45 angeschlossen, das als npn-Transistor ausgeführt ist. Über das elektrische Pulsschaltelement 45 ist die Induktivität 35 während des Vorhandenseins, also für die Dauer des Taktpulses 107, an den elektrischen Energiespeicher 33 und die Photozelle 27 anschließbar, also kurzzeitig bestrombar. Bei Vorhandensein des Taktpulses 107 wird die Induktivität 35 mit der elektrischen Energie 31 aufgeladen. Sobald das elektrische Pulsschaltelement 45 abschaltet, also nicht mehr Strom leitend ist, baut sich an der Induktivität 35 der entgegengesetzt wirkende Rückschlagimpuls 39 auf.

Da die an die Induktivität 35 schaltbare und im Folgenden näher beschriebene Messschaltung mittels des entgegengesetzt wirkenden Rückschlagimpulses 39, der in Form des spannungsbegrenzten Pulses 11 dort anliegt, versorgt wird, ist die Symbolisierung mit dem Masse-Symbol in Fig. 3 für die elektrische Energiequelle 9 scheinbar umgekehrt als üblich. Dadurch wird deutlich, dass die eigentliche Stromversorgung zum Messen und Signalisieren des Zustandes 3 des Wachstumssubstrates 5 jeweils für die Dauer des spannungsbegrenzten Pulses 11 lediglich durch die Induktivität 35 gewährleistet wird und damit auf besonders Strom sparende Art und Weise vorteilhaft möglich ist. Während der Taktpausen des Taktpulses 107 sind die Photozelle 27 sowie der elektrische Energiespeicher 33 mittels des Pulsschaltelementes 45 von der übrigen Mess- und Signalisierungsschaltung abgetrennt.

Mittels der Induktivität 35 ist das Messfilter 13 mit einer Sprungfunktion, also mit dem spannungsbegrenzten Puls 11 bestrombar. Das Messfilter 13 weist die Elektroden 69 und 71, also die Kapazität 67 auf, wobei die Kapazität 67 beziehungsweise deren zweite Elektrode 71 in dem vorliegenden Ausführungsbeispiel auf Masse bezogen ist. Denkbar ist auch eine Variation der Schaltungsanordnung 1, bei der die Kapazität 67 auf ein anderes neutrales Potential der Schaltung bezogen ist, beispielsweise die Versorgungsspannung. Außerdem weist das Messfilter 13 den Messwiderstand 115 auf. Der Messwiderstand 115 weist einen Widerstand von bevorzugt 100 kΩ auf, insbesondere zwischen 4,7 kΩ und 470 kΩ, insbesondere zwischen 90 kΩ und 110 kΩ. Die Auslegung des Messwiderstandes 115 kann variiert werden. Ebenso können eine Größe und ein Abstand sowie eine Positionierung der Elektroden 69 und 71 variiert werden. Dadurch kann das Übertragungsverhalten des Messfilters 13 beeinflusst werden. Insbesondere kann die Kapazität 67, insbesondere für einen definierten Zustand des Wachstumssubstrats 5, Werte zwischen 30 und 40 pF aufweisen. Vergleichsweise große oder größere Werte bedeuten, dass bereits bei einer vergleichsweise geringen Feuchtigkeitszufuhr "in Ordnung" signalisiert werden kann, und umgekehrt. So ist eine Auslegung für unterschiedliche Pflanzen möglich.

Außerdem ist es gemäß einer Alternative denkbar, die Elektroden 69 und 71 nicht auf Masse, sondern in Reihe zu dem Messwiderstand 115 zu schalten. In diesem Fall ergibt sich das Übertragungsverhalten eines Hochpasses und in dem in Figur 3 gezeichneten Fall eines Tiefpasses. In beiden Fällen kann dadurch ein zweiter Schalteingang 63 eines zweiten elektrischen Schaltelements 59 in der Signalisierungssteuerung 81 angesteuert werden. Das zweite elektrische Schaltelement 59 ist ebenfalls als npn-Transistor realisiert. Das zweite elektrische Schaltelement 59 ist in einen zweiten Parallelzweig 51 eines Vierpols 47 geschaltet. Der zweite Parallelzweig 51 weist die zweite Leuchtdiode 55 und dieser nachgeschaltet das zweite elektrische Schaltelement 59 auf und ist an die Induktivität 35 angeschlossen. Der Vierpol 47 weist außerdem einen ersten Parallelzweig 49 auf, der dem zweiten Parallelzweig 51 parallel geschaltet ist. Der erste Parallelzweig 49 ist also ebenfalls an die Induktivität 35 angeschlossen und weist die erste Leuchtdiode 53 und dieser nachgeschaltet das erste elektrische Schaltelement 57 auf, das ebenfalls als npn-Transistor ausgeführt ist. Die zwei an die Induktivität 35 angeschlossenen Parallelzweige 49 und 51 sind mittels eines Eingangswiderstandes 117 miteinander verbunden. Der Eingangswiderstand 117 verbindet die Kathode der zweiten Leuchtdiode 55 mit einem ersten Schalteingang 61 des ersten Schaltelements 57, also mit der Basis des npn-Transistors des ersten Schaltelements 57.

Es ist ersichtlich, dass der Vierpol 47 einen von einem Messausgang 65 des Messfilters 13, also der elektrischen Ausgangsgröße 17 bzw. einer entsprechenden Ausgangsspannung des Messfilters 13 gesteuerten Schwellwertschalter darstellt, wobei entweder die erste Leuchtdiode 53 oder die zweite Leuchtdiode 55 bestromt wird. Vorteilhaft ist also stets eine der Leuchtdioden 53 oder 55 bestromt und stellt damit die Spannungsbegrenzung des Rückschlagimpulses 39 zum Herstellen des spannungsbegrenzten Pulses 11 sicher. Sofern die Ausgangsspannung des Messfilters 13 unterhalb einer Schaltschwelle des Schwellwertschalters liegt, wird das zweite elektrische Schaltelement 59 nicht durchsteuert. In diesem Fall liegt über die Leuchtdiode 55 und den Eingangswiderstand 117 eine ausreichende Spannung an der Basis des ersten elektrischen Schaltelementes 57 an, sodass dieses durchschaltet und die erste Leuchtdiode 53 aufleuchtet.

Ein entsprechender Basisstrom des ersten elektrischen Schaltelements 57 kann also über die zweite Leuchtdiode 55 und den Eingangswiderstand 117 geführt werden. Dieser Basisstrom ist bevorzugt so gering, insbesondere durch eine entsprechende Auslegung des Eingangswiderstands 117, dass diese dabei nicht aufleuchtet. Um ein Aufleuchten der zweiten Leuchtdiode 55 beim Durchschalten des ersten elektrischen Schaltelementes 57 noch sicherer zu vermeiden, ist der zweiten Leuchtdiode 55 optional ein Widerstand 131 parallel geschaltet, beispielsweise von 100 kΩ.

Für den Fall, dass die Ausgangsspannung, also die elektrische Ausgangsgröße 17 des Messfilters 13, oberhalb der Schaltschwelle des Schwellwertschalters liegt, wird das zweite elektrische Schaltelement 59 durchgeschaltet, sodass die rote Leuchtdiode 55 aufleuchtet. Dabei wird über den Eingangswiderstand 117 der erste Schalteingang 61, also die Basis des Transistors des ersten elektrischen Schaltelements 57, auf Masse geschaltet, sodass dieses sperrt, also ausschließlich die rote Leuchtdiode 55 aufleuchtet.

Der durch den Vierpol 47 gebildete Schwellwertschalter weist also im Vergleich zu üblichen Schwellwertschaltern, die auch als Schmitt-Trigger bekannt sind, die Besonderheit auf, dass etwaige Verlustleistungen zum Erzielen des Schaltergebnisses direkt entweder in der Leuchtdiode 53 oder 55 anfallen, diese Verlustleistungen zum Signalisieren und gleichzeitig zum Spannungsbegrenzen - für die exakte Messung - verwendet werden und kein elektrischer Schaltausgang vorgesehen ist, da das Schaltergebnis mittels der Verlustleistung im Sinne eines optischen Ausgangs des Schwellwertschalters mittels der Leuchtdioden 53 und/oder 55 optisch signalisiert wird.

In der in Fig. 3A gezeigten Variante bildet das Messfilter 13 einen Tiefpass, sodass bei ausreichender Feuchtigkeit des Wachstumssubstrates 5 die Ausgangsspannung, also die elektrische Ausgangsgröße 17 aufgrund der vergleichsweise großen Kapazität 67 an den Elektroden 69 und 71 so langsam ansteigt, dass diese während des gesamten spannungsbegrenzten Pulses 11 nicht zum Durchschalten des zweiten elektrischen Schaltelements 59 genügt. Vorteilhaft wird also das erste Blitzsignal 21 zum Signalisieren des Zustandes 3 als in Ordnung wiedergegeben. Für den Fall eines zu trockenen Wachstumssubstrates 5 ergibt sich eine sehr geringe Kapazität 67 an den Elektroden 69 und 71, sodass die Spannung bzw. die elektrische Ausgangsgröße 17 des Messfilters 13 sehr schnell ansteigt und noch während der Dauer des spannungsbegrenzten Pulses 11 durchschaltet, sodass die zweite rote Leuchtdiode 55 aufleuchtet. Dabei ist der Anstieg so schnell, dass die erste Leuchtdiode 53 nicht oder zumindest nur unwesentlich, insbesondere quasi nicht optisch wahrnehmbar, angesteuert wird. Außerdem ist es denkbar, dass die elektrische Ausgangsgröße 17 während des spannungsbegrenzten Pulses 11 die Schaltschwelle des Schwellwertschalters überschreitet, sodass nacheinander zuerst die erste grüne LED 53 und dann die zweite rote LED 55 aufleuchten, beispielsweise zum Signalisieren des optimalen Zustandes 3 des Wachstumssubstrates 5.

Es ist ersichtlich, dass die in Figur 2 beschriebene Bedingung 109 der Signalisierungssteuerung 81 einen Schwellwert für die eigentlich zu messenden physikalische Größe 79 vorgibt. Dies wird durch eine Auswertung mittels der Schaltschwelle des Schwellwertschalters des Vierpols 47 und zusätzlich des zeitlichen Übertragungsverhaltens des Messfilters 13 erzielt. Die Bedingung 109 für eine ausreichende Feuchtigkeit ist dann erfüllt und wird entsprechend signalisiert, wenn die elektrische Ausgangsgröße 17 die Schaltschwelle unterschreitet und während der gesamten Dauer des spannungsbegrenzten Pulses 11 auch unterschritten bleibt. Für den Fall, dass die Schaltschwelle noch während des spannungsbegrenzten Pulses 11 überschritten wird, können beide Leuchtdioden 53 und 55 zum Signalisieren des optimalen Zustands nacheinander aufblitzen oder, falls das Überschreiten derart schnell erfolgt, dass die erste Leuchtdiode 53 nicht anspricht, also nicht sichtbar aufblitzt, kann dadurch nur die zweite Leuchtdiode 55 zum Signalisieren einer nicht ausreichenden Feuchtigkeit aufblitzen.

Fig. 3B zeigt eine Detailansicht einer möglichen Ausgestaltung des Messwiderstandes 115. Der Messwiderstand 115 kann durch einen veränderlichen und/oder einstellbaren Widerstand 119 ersetzt oder mit diesem in Reihe und/oder parallel geschaltet ergänzt werden. Wie in Fig. 3B dargestellt, ist der veränderliche/einstellbare Widerstand 119 in insgesamt drei Stufen schaltbar, insbesondere in zumindest zwei Stufen. Alternativ ist es denkbar, den veränderlichen/einstellbaren Widerstand 119 stufenlos veränderlich auszuführen, beispielsweise in Form eines Potentiometers. Vorteilhaft kann durch ein Einstellen des veränderlichen/einstellbaren Widerstands 119 das Übertragungsverhalten des Messfilters 13 der Messschaltung 105 verändert werden. Vorteilhaft kann dadurch ein Mess- und Signalisierungsverhalten der Schaltungsanordnung 1 der Messvorrichtung 99 angepasst werden, beispielsweise an einen Wasser- und Feuchtigkeitsbedarf unterschiedlicher in das Wachstumssubstrat 5 einpflanzbarer Pflanzen.

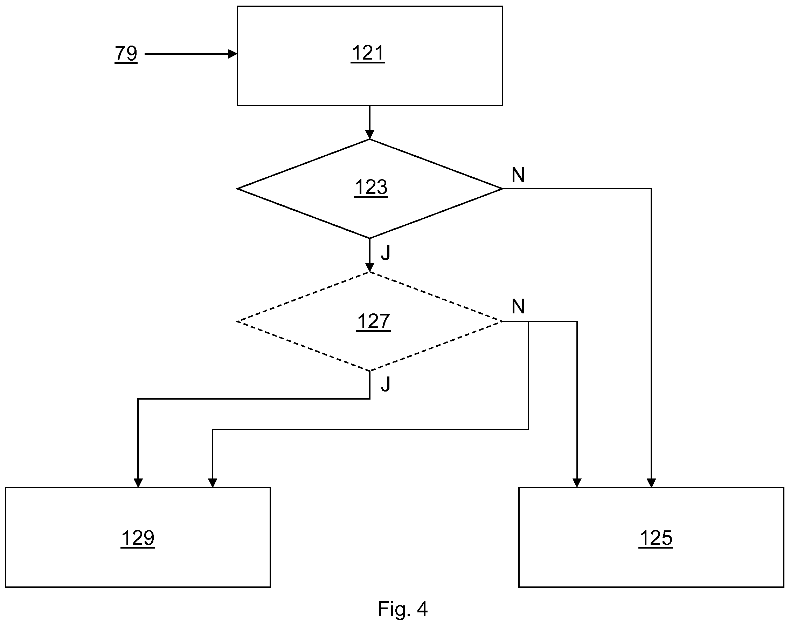

Fig. 4 zeigt ein Ablaufdiagramm eines Verfahrens zum Signalisieren eines Zustandes 3 eines Wachstumssubstrats 5 für eine Pflanze 7. Im Folgenden wird anhand der Fig. 4 ein insbesondere mittels der in den Fig. 1 - 3 näher erläuterten Messvorrichtung 99 durchführbares Verfahren erläutert.

In einem ersten Schritt 121 erfolgt eine Messung einer physikalischen Größe 79 des Wachstumssubstrates 5. Die gemessene physikalische Größe 79 wird, insbesondere in Form der Auswertung der elektrischen Ausgangsgröße 17 des Messfilters 13, mit einer ersten Schwelle 123 verglichen. Falls diese erste Schwelle 123 nicht überschritten ist, verzweigt das Verfahren in einen zweiten Schritt 125. Bei dem zweiten Schritt 125 wird ein optisch wahrnehmbares Blitzsignal wiedergegeben. Dies wird so lange wiedergegeben, wie die erste Schwelle 123 nicht überschritten ist. Unter so lange wiedergeben kann verstanden werden, dass das Verfahren zyklisch ausgeführt wird und mit einem bestimmten Zeitabstand, beispielsweise zwischen 2 und 5 s sich stets wiederholend ein kurzer Blitz wiedergegeben wird. Falls die erste Schwelle 123 überschritten ist, wird gemäß einer bevorzugten Ausführungsform, die nicht in Fig. 4 dargestellt ist, kein Blitzsignal wiedergegeben.

Gemäß einer weiteren, ebenfalls nicht in Figur 4 dargestellten Alternative, wird ein von diesem unterscheidbares Blitzsignal wiedergegeben.

Gemäß der Darstellung der Fig. 4 ist jedoch eine zweite Schwelle 127 als weitere mögliche Ausführungsform vorgegeben. Falls die erste Schwelle 123 überschritten ist, wird die zweite Schwelle 127 geprüft. Falls die erste Schwelle 123 überschritten ist und die zweite Schwelle 127 nicht überschritten ist, verzweigt das Verfahren ebenfalls zum zweiten Schritt 125 und zusätzlich zu einem dritten Schritt 129. Während des dritten Schrittes 129 wird ein weiteres optisches Blitzsignal, insbesondere das erste optische Blitzsignal 21 gleichzeitig mit dem zweiten Blitzsignal 23 wiedergegeben. Für den Fall, dass sowohl die erste Schwelle 123 als auch die zweite Schwelle 127 überschritten sind, wird ausschließlich der dritte Schritt 129 durchgeführt, also insbesondere lediglich das erste Blitzsignal 21 wiedergegeben.

Die Definition der Schwellen 123, 127 kann variiert werden, insbesondere ist es möglich anstatt einem Unterschreiten ein Überschreiten abzuprüfen, die Schwellen 123, 127 auf die zu messende physikalische Größe 79 zu beziehen oder auf eine in der Steuerkette nachgeschaltete abhängige Größe und/oder vor dem Abprüfen der Schwellen 123, 127 eine Invertierung vorzunehmen, und die Signalisierung entsprechend anzupassen.

Vorteilhaft kann eine Überwachung des Wachstumssubstrates 5 für die Pflanze 7 auf eine optimale Bodenfeuchte erfolgen. Insbesondere kann eine Man-in-the-Loop-Regelung realisiert werden. Vorteilhaft kann der Zustand 3 des Wachstumssubstrates 5 auf besonders leicht und gut wahrnehmbare Art und Weise, nämlich durch die Blitzsignale 21 und/oder 23 sowie auf besonders energiesparsame Art und Weise erfolgen.

Vorteilhaft ist es möglich, zumindest eine der Leuchtdioden 53, 55 nachts bzw. während einer schwachen Beleuchtung mit einer reduzierten Helligkeit zu betreiben, insbesondere mit der Farbe grün zum Signalisieren einer ausreichenden Feuchtigkeit.

Die Messvorrichtung 99 ist insbesondere stabförmig ausgeführt, sodass diese auf einfache Art und Weise in das Wachstumssubstrat 5 zumindest teilweise eingebracht werden kann.

Vorteilhaft ist es möglich, zumindest eine Eigenschaft zumindest eines der Blitzsignale 21, 23 zu verändern, um damit die auf die Pflanze 7 eintreffende Lichtmenge zusätzlich zu signalisieren. Dies kann insbesondere ebenfalls mittels des Dimmers 111 oder einer ähnlichen, durch eine Ausgangsspannung der Photozelle 27 gesteuerte Vorrichtung erfolgen. Insbesondere kann ein Abstand zwischen zwei Blitzen verändert werden, wo beispielsweise ein Abstand von 2 s signalisiert, dass viel Licht vorhanden ist und ein Abstand von 5 s zeigt, dass eine geringe Lichtmenge auf die Pflanze 7 trifft.

Mittels des Dimmers 111 kann vorteilhaft ein vergleichsweise intensives Aufblitzen des ersten Blitzsignals 21 und/oder des zweiten Blitzsignals 23 in ein längeres und/oder schwächeres Aufleuchten und/oder mit einem optischen Ausklingen heruntergeregelt werden. Diese Art der Signalisierung kann vorteilhaft als angenehm und nicht störend wahrgenommen werden. Insbesondere ist es möglich, eine solche Dimmung für die zweite Leuchtdiode 55 nicht vorzusehen, um ein Erfordernis eines Gießens der Pflanze 7 auch bei Nacht eindringlich zu signalisieren. Gemäß einer weiteren Alternative ist es möglich, insbesondere parallel zu der zweiten roten Leuchtdiode 55 das akustische Signal 89 wiederzugeben.

Vorteilhaft ergibt sich aufgrund der möglichen Ausgestaltung der elektrischen Energiequelle 9 mit der Induktivität 35 und einem als Kondensator ausgestalteten zusätzlichen elektrischen Energiespeicher 33 eine sehr hohe, fast unbegrenzte Lebensdauer. Vorteilhaft sind die zu speichernden Energiemengen vergleichsweise gering, sodass ein Kondensator verwendet werden kann. Insbesondere genügt ein Durchschnittsstrom von lediglich 2 bis 3 µA bei einer Spannung von 0,7 bis 2,5 V.

Vorteilhaft kann auch bei starken Schwankungen der Betriebsspannung, die von dem elektrischen Energiespeicher 33 und/oder der Photozelle 27 geliefert werden können, beispielsweise auch bei Spannungen von nur 0,6 bis 0,8 V durch die Spannungserhöhung der Induktivität 35 eine korrekte Messung erfolgen. Im umgekehrten Fall, beispielsweise bei einer vollen Beleuchtung der Photozelle 27, also bei einer vergleichsweise hohen Versorgungsspannung steht aufgrund der Induktivität 35 zusammenwirkend mit der Spannungsbegrenzung durch die Diodenvorrichtung 25 ebenfalls die für die Messung erforderliche konstante Spannung zur Verfügung. Vorteilhaft erfolgt die eigentliche Messung nur während der Dauer des spannungsbegrenzten Pulses 11, was einen niedrigen Energieverbrauch bedingt. Außerdem kann der Pulsgenerator 43 der Pulssteuerung 41 mit sehr niedrigen Strömen, beispielsweise ≤ 300 nA, betrieben werden. Dabei erfolgt die eigentliche Messung während sehr kurzen Zeiträumen, beispielsweise während zweihundert µs, wobei in diesen Zeiträumen auch gleichzeitig die Signalisierung erfolgt. Aufgrund der Spannungsbegrenzung können auch sehr hohe Spannungen des Rückschlagimpulses 39, beispielsweise von bis zu 60 V, verarbeitet werden. Entsprechend überschüssige elektrische Energie 31 wird vorteilhaft zum Signalisieren verwendet, also zum Erzeugen eines der Blitzsignale 21 oder 23. Ein etwaiger Stromverbrauch bzw. Energieverbrauch der Spannungsregelung geht also nicht verloren und wird unmittelbar in Lichtenergie umgesetzt. Vorteilhaft erfolgt dadurch auch eine von der Versorgungsspannung unabhängige Detektion bzw. Messung des Zustandes 3 bzw. der physikalischen Größe 79 des Wachstumssubstrats 5.

Die Elektroden 69 und 71 können aus einer Kupferbeschichtung der Leiterplatte 103 bestehen. Um Korrosion zu vermeiden, sind insbesondere beide durch den Schutzlack 73 geschützt. Vorteilhaft kann dies insbesondere ein üblicherweise verwendeter Lötstopplack sein.

Die erste Elektrode 69 ist an dem zweiten Schalteingang 63 des zweiten elektrischen Schaltelements 59 angeschlossen. Die zweite Elektrode 71 ist an ein neutrales Potenzial der Schaltungsanordnung 1 angeschlossen. Gemäß der Darstellung der Fig. 3A ist dies die Masse. Gemäß einer Alternative kann dies jedoch auch die Versorgungsspannung sein. Bei trockenem Wachstumssubstrat 5 entsteht keine wesentliche Kapazität 67 zwischen den Elektroden 69 und 71. Somit wird der spannungsbegrenzte Puls 11 nach dem Messwiderstand 115 kaum beeinflusst. Das zweite elektrische Schaltelement 59 schaltet durch und die zweite Leuchtdiode 55 blitzt auf. Bei feuchtem Wachstumssubstrat 5 bildet sich zwischen den Elektroden 69 und 71 eine beträchtliche Kapazität 67 aus und bildet zusammen mit dem Messwiderstand 115 den vorab beschriebenen Tiefpass. Dadurch wird der spannungsbegrenzte Puls 111 so weit gedämpft, dass die Spannung an der Basis des Transistors des zweiten elektrischen Schaltelements 59 nicht mehr ausreicht, um diesen durchzuschalten. Durch die Auslegung des Vierpols 47 als Schwellwertschalter leuchtet dadurch in Folge nicht die zweite Leuchtdiode 55, sondern die erste Leuchtdiode 53 auf.

In einem Übergangsbereich zwischen feuchtem Wachstumssubstrat 5 und trockenem Wachstumssubstrat 5 ist es möglich, dass entsprechend der Auslegung des Messwiderstandes 115 und der Elektroden 69 und 71 beide Leuchtdioden 53 und 55 während der Länge des spannungsbegrenzten Pulses 11 direkt nacheinander aufblitzen, sodass auch dieser Zustand 3 des Wachstumssubstrates 5, der beispielsweise einer optimalen Feuchte entspricht, signalisiert werden kann. Dazu schaltet der Schwellwertschalter des Vierpols 47 während der Pulsdauer einmal um.

Gemäß einer Alternative kann das Messfilter 13 als Hochpass ausgelegt werden. Dazu können die Elektroden 69 und 71 mit dem Messwiderstand 115 vertauscht werden. Die Ansteuerung des Vierpols 47 erfolgt dann invers zum vorab Beschriebenen, sodass auch gegebenenfalls die erste LED 53 und die zweite LED 55 in ihrer Farbwiedergabe zu tauschen wären, sodass auch dann eine grüne Signalisierung für eine ausreichende Feuchtigkeit des Wachstumssubstrates 5 steht.

### Bezugszeichenliste

| | | | |
|---|---|---|---|
| 1 | Schaltungsanordnung | 61 | erster Schalteingang |
| 3 | Zustand | 63 | zweiter Schalteingang |
| 5 | Wachstumssubstrat | 65 | Messausgang |
| 7 | Pflanze | 67 | Kapazität |
| 9 | elektrische Energiequelle | 69 | erste Elektrode |
| 11 | spannungsbegrenzter Puls | 71 | zweite Elektrode |
| 13 | Messfilter | 73 | Schutzlack |
| 15 | elektrische Wechselwirkung | 79 | physikalische Größe |
| 17 | elektrische Ausgangsgröße | 81 | Signalisierungssteuerung |
| 19 | optische Anzeigevorrichtung | 87 | Umgebungshelligkeit |
| 21 | erstes Blitzsignal | 89 | akustisches Signal |
| 23 | zweites Blitzsignal | 91 | elektrische Versorgungsspannung |
| 25 | Diodenvorrichtung | 99 | Messvorrichtung |
| 27 | Photozelle | 101 | Pflanzanordnung |
| 29 | Licht | 103 | Leiterplatte |
| 31 | elektrische Energie | 105 | Messschaltung |
| 33 | elektrischer Energiespeicher | 107 | Taktpuls |
| 35 | Induktivität | 109 | Bedingung |
| 37 | weiterer elektrischer Energiespeicher | 111 | Dimmer |
| 39 | Rückschlagimpuls | 113 | Person |
| 41 | Pulssteuerung | 115 | Messwiderstand |
| 43 | Pulsgenerator | 117 | Eingangswiderstand |
| 45 | elektrisches Pulsschaltelement | 119 | Widerstand |
| 47 | Vierpol | 121 | erster Schritt |
| 49 | erster Parallelzweig | 123 | erste Schwelle |
| 51 | zweiter Parallelzweig | 125 | zweiter Schritt |
| 53 | erste Leuchtdiode | 127 | zweite Schwelle |
| 55 | zweite Leuchtdiode | 129 | dritter Schritt |
| 57 | erstes elektrisches Schaltelement | 131 | Widerstand |
| 59 | zweites elektrisches Schaltelement | | |

## Patentansprüche

1. Verfahren zum Signalisieren eines Zustands (3) eines Wachstumssubstrats (5) für eine Pflanze (7), mit:
- Bereitstellen einer elektrischen Energiequelle (9),
- Bereitstellen einer von der elektrischen Energiequelle (9) mit einem Puls elektrisch versorgbaren Messschaltung (105),
- Kontinuierliches Signalisieren eines Zustands (3) des Wachstumssubstrats (5) durch eine Folge unterscheidbarer, optisch wahrnehmbarer Blitzsignale (21,23) mittels einer optischen Anzeigevorrichtung (19), **gekennzeichnet durch**:
- Herstellen einer elektrischen Wechselwirkung (15) mit dem Wachstumssubstrat (5) mittels einer in das Wachstumssubstrat (5) einbringbaren Kapazität (67) eines Messfilters (13) der Messschaltung (105), wobei eine Kapazität der einbringbaren Kapazität (67) von dem Zustand (3) des Wachstumssubstrats (5) und die elektrische Ausgangsgröße (17) des Messfilters (13) von der Kapazität der einbringbaren Kapazität (67) abhängen,
- Wandeln von Licht (29) in elektrische Energie (31) mittels wenigstens einer Photozelle (27) der elektrischen Energiequelle (9),
- Erzeugen des Pulses als spannungsbegrenzter Puls (11) mittels der elektrischen Energie (31) und
- elektrisches Versorgen der optischen Anzeigevorrichtung (19) zum kontinuierlichen Signalisieren des Zustands (3), einer der Messschaltung (105) nachgeschalteten Signalisierungssteuerung (81) zum Ansteuern der optischen Anzeigevorrichtung (19) und des Messfilters (13) zum Messen des Zustands (3) mittels des spannungsbegrenzten Pulses (11).

2. Verfahren nach Anspruch 1, mit:
- Messen einer bei einer Pflanze (7) und/oder dem Wachstumssubstrat (5) herrschenden Helligkeit,
- Steuern zumindest einer der folgenden Eigenschaften des Blitzsignals (21,23) in Abhängigkeit der gemessenen Helligkeit: Eine Leuchtdauer eines einzelnen Blitzes, eine Helligkeit eines einzelnen Blitzes, ein optisches Ausklingen oder Anschwellen eines einzelnen Blitzes, eine Dauer eines einzelnen Blitzes, ein zeitlicher Abstand zwischen zwei aufeinanderfolgenden Blitzen.

3. Verfahren nach Anspruch 1 oder 2, mit
- Messen einer vom Zustand (3) des Wachstumssubstrats (5) abhängigen physikalischen Größe (79) durch Bestimmung der Begrenzung der Spannung des das Blitzsignal hervorrufenden spannungsbegrenzten Pulses (11) durch eine das Blitzsignal erzeugende Leuchtdiode (LED).

4. Verfahren nach Anspruch 3, mit
- Speichern der von der wenigstens einen Photozelle (27) erzeugten Energie auf einem Energiespeicher (33), wobei die Spannung des Energiespeichers (33) kleiner gleich einer vom Energiespeicher (33) zu liefernden Versorgungsspannung der optischen Anzeigevorrichtung (19) ist.

5. Schaltungsanordnung (1) zum Signalisieren eines Zustands (3) eines Wachstumssubstrats (5) für eine Pflanze (7), mit:
- einer elektrischen Energiequelle (9),
- einer von der elektrischen Energiequelle (9) mit einem Puls elektrisch versorgbaren Messschaltung (105),
- einer optischen Anzeigevorrichtung (19), die zum kontinuierlichen Signalisieren des Zustands (3) durch eine Folge unterscheidbarer, optisch wahrnehmbarer Blitzsignale kurzzeitig einschaltbar ist,
- einer der Messschaltung (105) nachgeschalteten Signalisierungssteuerung (81) zum Ansteuern der optischen Anzeigevorrichtung (19),
**dadurch gekennzeichnet, dass** die Messschaltung (105) ein Messfilter (13) zum Herstellen einer elektrischen Wechselwirkung (15) mit dem Wachstumssubstrat (5) mittels einer in das Wachstumssubstrat (5) einbringbaren Kapazität (67) des Messfilters (13) aufweist, wobei eine Kapazität der einbringbaren Kapazität (67) von dem Zustand (3) des Wachstumssubstrats (5) und die elektrische Ausgangsgröße (17) des Messfilters (13) von der Kapazität der J Z einbringbaren Kapazität (67) abhängen, und dass die elektrische Energiequelle (9) wenigstens eine Photozelle (27) zum Wandeln des Lichts (29) in elektrische Energie (31) aufweist, wobei mittels der elektrischen Energie (31) der Puls als spannungsbegrenzter Puls (11) erzeugbar ist und wobei durch den spannungsbegrenzten Puls (11) das Messfilter (13), die Signalisierungssteuerung (81) und die optische Anzeigevorrichtung (19) mit der elektrischen Energie (31) versorgbar sind, wobei wenigstens erste und/oder zweite Blitzsignale (21,23) dauerhaft wiedergebbar sind.

6. Schaltungsanordnung nach Anspruch 5, wobei die elektrische Energiequelle (9) und die optische Anzeigevorrichtung (19) eine gemeinsame Diodenvorrichtung (25) aufweisen, mittels der gleichzeitig der Puls (11) spannungsbegrenzbar und die wenigstens ersten und/oder zweiten Blitzsignale (21,23) dauerhaft wiedergebbar sind.

7. Schaltungsanordnung nach Anspruch 5 oder 6, wobei die elektrische Energiequelle einen der wenigstens einen Photozelle (27) parallel geschalteten Energiespeicher (33) zum Zwischenspeichern der elektrischen Energie (31) aufweist.

8. Schaltungsanordnung nach Anspruch 7, wobei die Spannung des Energiespeichers (33) kleiner gleich einer vom Energiespeicher (33) zu liefernden Versorgungsspannung der optischen Anzeigevorrichtung (19) ist.

9. Schaltungsanordnung nach einem der Ansprüche 5 bis 8, wobei die elektrische Energiequelle (9) eine Induktivität (35) als weiteren elektrischen Energiespeicher (37) aufweist, mittels der zum Erzeugen des spannungsbegrenzten Pulses ein Rückschlagimpuls (39) erzeugbar ist.

10. Schaltungsanordnung nach Anspruch 9, wobei die elektrische Energiequelle (9) eine Pulssteuerung (41) aufweist, mittels der die Induktivität (35) zum Erzeugen des Rückschlagimpulses (39) kurzzeitig bestrombar ist.

11. Schaltungsanordnung nach einem der Ansprüche 5 bis 10, dazu eingerichtet, dass das Messfilter (13) und die optische Anzeigevorrichtung (19) in einer Zeit, in der kein spannungsbegrenzter Puls vorhanden ist, im Wesentlichen stromlos sind.

12. Schaltungsanordnung nach einem der Ansprüche 6 bis 11, wobei die optische Anzeigevorrichtung (19) einen durch den spannungsbegrenzten Puls (11) mit elektrischer Energie (31) versorgbaren Vierpol (47) aufweist, der in einem ersten Parallelzweig (49) eine erste Leuchtdiode (53) der Diodenvorrichtung (25) sowie ein erstes elektrisches Schaltelement (57) zum Schalten der ersten Leuchtdiode (53) und in einem zweiten Parallelzweig (51) eine zweite Leuchtdiode (55) der Diodenvorrichtung (25) sowie ein zweites elektrisches Schaltelement (59) zum Schalten der zweiten Leuchtdiode (55) aufweist, wobei ein erster Schalteingang (61) des ersten elektrischen Schaltelements (57) der zweiten Leuchtdiode (55) nachgeschaltet ist und ein zweiter Schalteingang (63) des zweiten elektrischen Schaltelements (59) einem Messausgang (65) des Messfilters (13) nachgeschaltet ist.

13. Schaltungsanordnung nach einem der Ansprüche 5 bis 12, aufweisend einen elektrischen Messwiderstand (115) und/oder einen einstellbaren elektrischen Widerstand (119), wobei der einbringbaren Kapazität (67) der elektrische Messwiderstand (115) und/oder der einstellbare elektrische Widerstand (119) vorgeschaltet ist/sind.

14. Messvorrichtung (99) mit einer Schaltungsanordnung (1) nach einem der Ansprüche 5 bis 13, aufweisend eine Leiterplatte (103) und wobei die Messvorrichtung (99) Elektroden (69) und (71) aufweist, die gegenüberliegend beabstandet auf der Leiterplatte (103) angebracht sind.

15. Messvorrichtung (99) nach Anspruch 14, wobei die Photozelle (27) eine ablesbare oder wahrnehmbare Botschaft aufweist, wobei bevorzugt die zumindest eine der oder die Leuchtdioden (53,55) benachbart zu oder auf gegenüberliegenden Seiten der ablesbaren oder wahrnehmbaren Botschaft der Photozelle (27) angeordnet ist/sind.

## Claims

1. A method for signalling a state (3) of a growth substrate (5) for a plant (7), having the following steps:
- providing an electrical energy source (9),
- providing a measuring circuit (105) that may be electrically supplied with a pulse by the electrical energy source (9),
- continuously signalling a state (3) of the growth substrate (5) by a sequence of distinguishable, optically perceivable flash signals (21, 23) by means of an optical display device (19),
**characterised by**:
- producing an electrical interaction (15) with the growth substrate (5) by means of a capacitor (67) of a measuring filter (13) of the measuring circuit (105), which capacitor is introducible into the growth substrate (5), a capacitance of the introducible capacitor (67) being dependent on the state (3) of the growth substrate (5), and the electrical output variable (17) of the measuring filter (13) being dependent on the capacitance of the introducible capacitor (67),
- converting light (29) into electrical energy (31) by means of at least one photocell (27) of the electrical energy source (9),
- producing the pulse as a voltage-limited pulse (11) by means of the electrical energy (31), and
- electrically supplying the optical display device (19) for continuously signalling the state (3), a signalling controller (81) arranged downstream of the measuring circuit (105) for controlling the optical display device (19), and the measuring filter (13) for measuring the state (3) by means of the voltage-limited pulse (11).

2. A method in accordance with claim 1, having the following steps:
- measuring a brightness prevailing at a plant (7) and/or the growth substrate (5),
- controlling at least one of the following properties of the flash signal (21, 23) depending on the measured brightness: a flash duration of an individual flash, a brightness of an individual flash, an optical fading or surging of an individual flash, a duration of an individual flash, a time interval between two successive flashes.

3. A method in accordance with claim 1 or 2, having the following steps:
- measuring a physical variable (79) dependent on the state (3) of the growth substrate (5) by determining the limit of the voltage of the voltage-limited pulse (11) causing the flash signal by a light-emitting diode (LED) producing the flash signal.

4. A method in accordance with claim 3, having the following steps:
- storing the energy produced by the at least one photocell (27) on an energy store (33), the voltage of the energy store (33) being less than or equal to a supply voltage of the optical display device (19) to be delivered by the energy store.

5. A circuit arrangement (1) for signalling a state (3) of a growth substrate (5) for a plant (7), having the following:
- an electrical energy source (9),
- a measuring circuit (105) that may be electrically supplied with a pulse by the electrical energy source (9),
- an optical display device (19), which may be switched on temporarily by a sequence of distinguishable, optically perceivable flash signals to continuously signal the state (3),
- a signalling controller (81) arranged downstream of the measuring circuit (105) for controlling the optical display device (19), **characterised in that** the measuring circuit (105) has a measuring filter (13) for producing an electrical interaction (15) with the growth substrate (5) by means of a capacitor (67) of the measuring filter (13), which capacitor is introducible into the growth substrate (5), a capacitance of the introducible capacitor (67) being dependent on the state (3) of the growth substrate (5), and the electrical output variable (17) of the measuring filter (13) being dependent on the capacitance of the introducible capacitor (67), and **in that** the electrical energy source (9) has at least one photocell (27) for converting the light (29) into electrical energy (31), the pulse being producible as a voltage-limited pulse (11) by means of the electrical energy (31), and the measuring filter (13), the signalling controller (81) and the optical display device (19) being suppliable with the electrical energy (31) by the voltage-limited pulse (11), at least first and/or second flash signals (21, 23) being sustainably reproducible.

6. A circuit arrangement in accordance with claim 5, the electrical energy source (9) and the optical display device (19) having a common diode device (25), by means of which the pulse (11) may be voltage-limited and, at the same time, the at least first and/or second flash signals (21, 23) are sustainably reproducible.

7. A circuit arrangement in accordance with claim 5 or 6, the electrical energy source having an energy store (33) connected in parallel with the at least one photocell (27) for temporarily storing the electrical energy (31).

8. A circuit arrangement in accordance with claim 7, the voltage of the energy store (33) being less than or equal to a supply voltage of the optical display device (19) to be delivered by the energy store (33).

9. A circuit arrangement in accordance with claims 5 to 8, the electrical energy source (9) having an inductor (35) as further electrical energy store (37), by means of which inductor a return pulse (39) is producible in order to produce the voltage-limited pulse.

10. The circuit arrangement in accordance with claim 9, the electrical energy source (9) having a pulse controller (41), by means of which the inductor (35) for producing the return pulse (39) is temporarily energisable.

11. A circuit arrangement in accordance with claims 5 to 10, arranged such that the measuring filter (13) and the optical display device (19) are substantially currentless for a time during which no voltage-limited pulse is provided.

12. A circuit arrangement in accordance with claims 6 to 11, the optical display device (19) having a quadripole (47) suppliable with electrical energy (31) by the voltage-limited pulse (11), which quadripole in a first parallel branch (49) has a first light-emitting diode (53) of the diode device (25) and a first electrical switch element (57) for switching the first light-emitting diode (53) and in a second parallel branch (51) has a second light-emitting diode (55) of the diode device (25) and a second electrical switch element (59) for switching the second light-emitting diode (55), a first switch input (61) of the first electrical switch element (57) being arranged downstream of the second light-emitting diode (55) and a second switch input (63) of the second electrical switch element (59) being arranged downstream of a measurement output (65) of the measuring filter (13).

13. A circuit arrangement in accordance with claims 5 to 12, having an electrical measuring resistor (115) and/or an adjustable electrical resistor (119), the electrical measuring resistor (115) and/or the adjustable electrical resistor (119) being arranged upstream of the introducible capacitor (67).

14. A measuring device (99) having a circuit arrangement (1) in accordance with claims 5 to 13, having a printed circuit board (103) and the measuring device (99) having electrodes (69) and (71), which are mounted oppositely and spaced apart on the printed circuit board (103).

15. A measuring device (99) in accordance with claim 14, the photocell (27) having a readable or perceivable message, at least one of the light-emitting diodes (53, 55) preferably being arranged adjacently to or on opposite sides of the readable or perceivable message of the photocell (27).

## Revendications

1. Procédé pour signaler l'état (3) d'un substrat de croissance (5) pour une plante (7), comportant :
- la mise à disposition d'une source d'énergie électrique (9),
- la mise à disposition d'un circuit de mesure (105) pouvant être alimenté électriquement par la source d'énergie électrique (9) avec une impulsion,
- le signalement continu d'un état (3) du substrat de croissance (5) à l'aide d'une suite de signaux lumineux (21,23) différenciables et perceptibles optiquement grâce à un dispositif d'affichage optique (19),
**caractérisé par** :
- la réalisation d'une interaction électrique (15) avec le substrat de croissance (5) au moyen d'une capacité (67) d'un filtre de mesure (13) du circuit de mesure (105) insérable dans le milieu de croissance (5), une capacité de la capacité insérable (67) dépendant de l'état (3) du substrat de croissance (5) et la grandeur de sortie électrique (17) du filtre de mesure (13) dépendant de la capacité de la capacité insérable (67),
- la transformation de la lumière (29) en énergie électrique (31) au moyen d'au moins une cellule photoélectrique (27) de la source d'énergie électrique (9),
- la production de l'impulsion en tant qu'impulsion limitée en tension (11) au moyen de l'énergie électrique (31) et
- l'alimentation électrique du dispositif d'affichage optique (19) pour le signalement continu de l'état (3), d'une commande de signalement (81) connectée en aval du circuit de mesure (105) pour la commande du dispositif d'affichage optique (19) et du filtre de mesure (13) pour la mesure de l'état (3) au moyen de l'impulsion limitée en tension (11).

2. Procédé selon la revendication 1, comportant
- la mesure d'une luminosité régnant au niveau d'une plante (7) et/ou du substrat de croissance (5),
- la commande d'au moins une des propriétés suivantes du signal lumineux (21,23) en fonction de la luminosité mesurée : une durée de rayonnement d'un flash individuel, une luminosité d'un flash individuel, une diminution ou une augmentation d'un flash individuel, une durée d'un flash individuel, un intervalle temporel entre deux flashs consécutifs.

3. Procédé selon la revendication 1 ou 2, comportant :
- la mesure d'une grandeur physique (79) dépendant de l'état (3) d'un substrat de croissance (5) par détermination de la limitation de la tension de l'impulsion (11) limitée en tension provoquant le signal lumineux à l'aide d'une diode électroluminescente (LED) produisant le signal lumineux.

4. Procédé selon la revendication 3, comportant
- le stockage de l'énergie produite par la ou les cellules photoélectriques (27) sur un dispositif de stockage d'énergie (33), la tension du dispositif de stockage d'énergie (33) étant inférieure ou égale à une tension d'alimentation du dispositif d'affichage (19) à fournir par le dispositif de stockage d'énergie (33).

5. Circuit (1) pour le signalement d'un état (3) d'un substrat de croissance (5) pour une plante (7), comportant :
- une source d'énergie électrique (9),
- un circuit de mesure (105) pouvant être alimenté électriquement par la source d'énergie électrique (9) avec une impulsion,
- un dispositif d'affichage optique (19) qui peut être mis en marche brièvement par une suite de signaux lumineux différenciables, perceptibles optiquement pour le signalement continu de l'état (3),
- une commande de signalement (81) connectée en aval du circuit de commande (105) pour la commande du dispositif d'affichage (19),
**caractérisé en ce que** le circuit de mesure (105) présente un filtre de mesure (13) pour la réalisation d'une interaction électrique (15) avec le substrat de croissance (5) au moyen d'une capacité (67) du filtre de mesure (13) insérable dans le substrat de croissance (5), une capacité de la capacité insérable (67) dépendant de l'état (3) du substrat de croissance (5) et la grandeur de sortie électrique (17) du filtre de mesure (13) dépendant de la capacité de la capacité insérable (67) et **en ce que** la source d'énergie électrique (9) présente au moins une cellule photoélectrique (27) pour la transformation de la lumière (29) en énergie électrique (31), l'impulsion pouvant être produite en tant qu'impulsion limitée en tension au moyen de l'énergie électrique (31), et le filtre de mesure (13), la commande de signalement (81) et le dispositif d'affichage optique (19) pouvant être alimentés par l'énergie électrique (31) à l'aide de l'impulsion limitée en tension (11), au moins des premiers et/ou deuxièmes signaux lumineux (21,23) étant reproductibles de façon permanente.

6. Circuit selon la revendication 5, dans lequel la source d'énergie électrique (9) et le dispositif d'affichage optique (19) présentent un dispositif à diodes (25) commun, au moyen duquel sont reproductibles de façon permanente en même temps l'impulsion (11) d'une manière limitée en tension et au moins les premiers et/ou deuxièmes signaux lumineux (21,23).

7. Circuit selon la revendication 5 ou 6, dans lequel la source d'énergie électrique présente un dispositif de stockage d'énergie (33) connecté en parallèle à la ou au(x) cellule(s) photoélectrique (27) pour le stockage intermédiaire de l'énergie électrique (31).

8. Circuit selon la revendication 7, dans lequel la tension du dispositif de stockage d'énergie (33) est inférieure ou égale à une tension d'alimentation du dispositif d'affichage optique (19) à fournir par le dispositif de stockage d'énergie (33).

9. Circuit selon l'une des revendications 5 à 8, dans lequel la source d'énergie électrique (9) présente une inductance (35) en tant que dispositif de stockage d'énergie électrique supplémentaire (37), au moyen de laquelle une impulsion de retour (39) peut être produite pour la production de l'impulsion limitée en tension.

10. Circuit selon la revendication 9, dans lequel la source d'énergie électrique (9) présente une commande d'impulsions (41), au moyen de laquelle l'inductance (35) pour la production de l'impulsion de retour (39) peut être alimentée brièvement.

11. Circuit selon l'une des revendications 5 à 10, agencé de telle sorte que le filtre de mesure (13) et le dispositif d'affichage optique (19) soient essentiellement dépourvus de courant pendant une période où il n'y a pas d'impulsion limitée en tension.

12. Circuit selon l'une des revendications 6 à 11, dans lequel le dispositif d'affichage optique (19) présente un quadripôle (47) pouvant être alimenté par l'impulsion limitée en tension (11) en énergie électrique (31), lequel quadripôle présente, dans une première dérivation parallèle (49), une première diode électroluminescente (53) du dispositif à diodes (25) ainsi qu'un premier élément de commutation électrique (57) pour la commutation vers la première diode électroluminescente (53) et, dans une deuxième dérivation parallèle, une deuxième diode électroluminescente (55) du dispositif à diodes (25) ainsi qu'un deuxième élément de commutation électrique (59) pour la commutation vers la deuxième diode électroluminescente (55), une première entrée de commutation (61) du premier élément de commutation électrique (57) étant connectée en aval de la deuxième diode électroluminescente (55) et une deuxième entrée de commutation (63) du deuxième élément de commutation électrique (59) étant connectée en aval d'une sortie de mesure (65) du filtre de mesure (13).

13. Circuit selon l'une des revendications 5 à 12, présentant une résistance électrique de mesure (115) et/ou une résistance électrique (119) réglable, la résistance électrique de mesure (115) et/ou la résistance électrique réglable (119) étant connectée(s) en amont de la capacité insérable (67).

14. Dispositif de mesure (99) comportant un circuit (1) selon l'une des revendications 5 à 13, comprenant un circuit imprimé (103) et dans lequel le dispositif de mesure (99) présente des électrodes (69) et (71) qui sont branchées face à face de manière espacée sur le circuit imprimé (103).

15. Dispositif de mesure (99) selon la revendication 14, dans lequel la cellule photoélectrique (27) présente un message lisible ou perceptible, de préférence la ou l'une au moins des diodes électroluminescentes (53,55) étant disposé(s) de manière adjacente au, ou sur des côtés opposés du message lisible ou perceptible de la cellule photoélectrique (27).
